# EUROPEAN PATENT APPLICATION

(11) **EP 2 377 953 A1**
(43) Date of publication of application: **19.10.2011**
(21) Application number: 11167754.8
(22) Date of filing: 25.06.2007
(51) Int. Cl.: C12Q 1/68, C12N 9/02

(54) **DPYD gene variants and use thereof**

(30) Priority: 23.06.2006 US 816090 P; 26.10.2006 US 863104 P
(62) Divisional of application: 07799007.5
(71) Applicant: MYRIAD GENETICS, INC., Salt Lake City, Utah 84108 (US)
(72) Inventor: Wagner, Susanne, Salt Lake City, UT 84108 (US); Potter, Jennifer, Salt Lake City, UT 84106 (US)
(74) Representative: Vossius & Partner

(57) **Abstract**

Variants in DPYD gene are disclosed which can result in abnormal synthesis of DPD proteins and alteration of DPD activities. The invention provides methods for detecting the newly discovered genetic variants. The DPD genetic variants of the invention can be used as biomarkers in predicting toxicity to 5-FU and other drugs metabolized by the DPD enzyme.

## Description

### CROSS-REFERENCE TO RELATED APPLICATIONS

This application claims priority to U.S. provisional application Serial Nos. 60/816,090, filed June 23, 2006, and 60/863,104, filed October 26, 2006, both of which are hereby incorporated by reference in their entireties.

### FIELD OF THE INVENTION

This invention generally relates to pharmacogenetics, particularly to the identification of genetic variants that are associated with human dehydropyrimidine dehydrogenase, and methods of using the identified variants.

### BRIEF DESCRIPTION OF THE SEQUENCE LISTING

SEQ ID NO:1 refers to a human dihydropyrimidine dehydrogenase mRNA sequence. SEQ ID NO:2 refers to a human dihydropyrimidine dehydrogenase protein sequence encoded by the nucleotide sequence of SEQ ID NO:1.

### BACKGROUND OF THE INVENTION

The human dihydropyrimidine dehydrogenase gene (*"DPYD"*) encodes a protein (DPD) that catalyzes the first and rate-limiting step in pyrimidine metabolism. DPD deficiency is associated with potentially life-threatening toxicity to 5-fluorouracil (5-FU) and drugs that are metabolized by the enzyme encoded by *DPYD.*

DPD (EC 1.3.1.2) is the principal enzyme involved in the degradation of 5-FU, a cancer drug, which is thought to act by inhibiting thymidylate synthase (TS). See *e.g*., Heggie et al. (1987) Cancer Res. 47: 2203-2206 and Diasio et al. (1989) Clin. Pharmacokinet. 16: 215-27. A recently approved cancer drug that is metabolized by DPD is capecitabine (Xeloda^{™}), which also has the potential to cause severe toxicity in patients with DPD deficiency. See Saif et al. (2006) Clin. Colorectal Cancer (5):359-62, The drug label for capecitabine indicates that the drug is contraindicated for patients with DPD deficiency. Capecitabine is a 5-FU prodrug.

The level of DPD activity is known to affect the efficacy of 5-FU treatments since 5-FU plasma levels are inversely correlated with the level of DPD activity. See, Iigo et al. (1988) Biochem. Pharm. 37: 1609-1613; Goldberg et al. (1988) Br. J. Cancer 57: 186-189; Harris et al. (1991) Cancer (Phila.) 68: 499-501; Fleming et al. (1991) Proc. Am. SAc. Cancer Res. 32: 179. In turn, the efficacy of 5-FU treatment of cancer is correlated with plasma levels of 5-FU.

DPD is the initial and rate limiting enzyme in uracil and thymine catabolism, leading to the formation of β-alanine and β-aminobutyric acid, respectively. See *e.g.*, Wasternack et al. (1980) Pharm. Ther. 8: 629-665. DPD deficiency is associated with inherited disorders of pyrimidine metabolism, clinically termed thymine-uraciluria. See, Bakkeren et al. (1984) Clin. Chim. Acta. 140: 247-256. Some clinical symptoms of DPD deficiency include nonspecific cerebral dysfunction, psychomotor retardation, convulsions, and epileptic conditions. See *e.g.,* Berger et al. (1984) Clin. Chim. Acta 141: 227-234; Wadman et al. (1985) Adv. Exp. Med. Biol. 165A: 109-114; Wulcken et al. (1985) J. Inherit. Metab. Dis. 8 (Suppl. 2): 115-116; van Gennip et al. (1989) Adv. Exp. Med. Biol. 253A: 111-118; Brockstedt et al. (1990) J. Inherit. Metab. Dis. 12: 121-124; and Duran et al. (1991) J. Inherit. Metab. Dis. 14: 367-370. Patients having DPD deficiency have an almost complete absence of DPD activity in fibroblasts and in lymphocytes (Piper et al. (1980) Biochim. Biophys. Acta 633: 400-409). Large accumulations of uracil and thymine are observed in the cerebrospinal fluid and urine of these patients (see *e.g*., Fleming et al. (1992) Cancer Res. 52: 2899-2902).

Given that DPD deficiency is associated with life-threatening toxicity, there is a need for the identification of novel *DPYD* variants.

### SUMMARY OF THE INVENTION

The present invention is based on the discovery of a number of genetic mutations in the *DPYD* gene. The nucleotide and amino acid variants are summarized below in the Detailed Description of the Invention. The variants can be deleterious and cause significant alterations in the structure, biochemical activity, and/or expression level of the human DPD protein. Thus, the nucleotide (and amino acid) variants can be useful in genetic testing as markers for the prediction of toxicity to drugs which are metabolized by the DPD enzyme and for prediction of efficacy of such drugs.

Accordingly, in a first aspect of the present invention, an isolated human *DPYD* nucleic acid is provided containing at least one of the newly discovered genetic polymorphic variants as summarized in Table 1 below. The present invention also encompasses an isolated oligonucleotide having a contiguous span of at least 18, preferably from 18 to 50 nucleotides of the sequence of a human *DPYD* gene, wherein the contiguous span encompasses and contains a nucleotide variant selected from those in Table 1. One example of such a *DPYD* nucleic is a PCR amplicon having the variant. Another example is a preparation for high pressure liquid chromatography analysis (HPLC) having a *DPYD* nucleic acid containing one of the *DPYD* variants of the invention.

DNA microchips are also provided comprising an isolated *DPYD* gene or an isolated oligonucleotide according to the present invention. In accordance with another aspect of the invention, an isolated DPD protein or a fragment thereof is provided having an amino acid variant selected from those in Table 1.

In accordance with another aspect of the invention, a deleterious DPD mutant protein or gene is provided herein.

The present invention also provides an isolated antibody specifically immunoreactive with a DPD protein variant of the present invention.

In accordance with yet another aspect of the present invention, a method is provided for genotyping the *DPYD* gene of an individual by determining whether the individual has a genetic variant or an amino acid variant provided in accordance with the present invention. In addition, the present invention also provides a method for predicting in an individual susceptible to toxicity to a drug that is metabolized by the DPD enzyme, *e.g*., 5-FU. The method comprises the step of detecting in the individual the presence or absence of a genetic variant or amino acid variant provided according to the present invention.

In accordance with yet another aspect of the invention, a detection kit is provided for detecting, in an individual, an elevated susceptibility to toxicity to a drug that is metabolized by the DPD enzyme, *e.g*., a fluoropyrimidine like 5-FU or capecitabine. In a specific embodiment, the kit is used in determining susceptible to toxicity to a drug that is metabolized by the DPD enzyme, *e.g*., 5-FU. The kit may include, in a carrier or confined compartment, any nucleic acid probes or primers, or antibodies useful for detecting the nucleotide variants or amino acid variants of the present invention as described above. The kit can also include other reagents such as DNA polymerase, buffers, nucleotides and others that can be used in the method of detecting the variants according to this invention. In addition, the kit preferably also contains instructions for using the kit.

In accordance with yet another aspect of the present invention, a method is provided for determining whether an individual has a haplotype, amino acid variant, and/or genetic marker according to the present invention, that is associated altered *DPYD* activity levels. In addition, the present invention also provides a method for predicting in an individual susceptible to toxicity to a drug that is metabolized by the DPD enzyme, *e.g*., 5-FU or capecitabine. The method comprises the step of detecting in the individual the presence or absence of a genetic variant, amino acid variant, and/or haplotype provided according to the present invention.

The foregoing and other advantages and features of the invention, and the manner in which the same are accomplished, will become more readily apparent upon consideration of the following detailed description of the invention taken in conjunction with the accompanying examples and drawings, which illustrate preferred and exemplary embodiments.

### DETAILED DESCRIPTION OF THE INVENTION

### 1. Definition

The terms "genetic variant" and "nucleotide variant" are used herein interchangeably to refer to changes or alterations to the reference human *DPYD* gene or cDNA sequence at a particular locus, including, but not limited to, nucleotide base deletions, insertions, inversions, and substitutions in the coding and noncoding regions. Deletions may be of a single nucleotide base, a portion or a region of the nucleotide sequence of the gene, or of the entire gene sequence. Insertions may be of one or more nucleotide bases. The "genetic variant" or "nucleotide variants" may occur in transcriptional regulatory regions, untranslated regions of mRNA, exons, introns, or exon/intron junctions. The "genetic variant" or "nucleotide variants" may or may not result in stop codons, frame shifts, deletions of amino acids, altered gene transcript splice forms or altered amino acid sequence.

The term "allele" or "gene allele" is used herein to refer generally to a naturally occurring gene having a reference sequence or a gene containing a specific nucleotide variant.

As used herein, "haplotype" is a combination of genetic (nucleotide) variants in a region of an mRNA or a genomic DNA on a chromosome found in an individual. Thus, a haplotype includes a number of genetically linked polymorphic variants which are typically inherited together as a unit.

As used herein, the term "amino acid variant" is used to refer to an amino acid change to a reference human DPD protein sequence resulting from "genetic variants" or "nucleotide variants" to the reference human gene encoding the reference DPD protein. The term "amino acid variant" is intended to encompass not only single amino acid substitutions, but also amino acid deletions, insertions, and other significant changes of amino acid sequence in the reference DPD protein.

The term "genotype" as used herein means the nucleotide characters at a particular nucleotide variant marker (or locus) in either one allele or both alleles of a gene (or a particular chromosome region). With respect to a particular nucleotide position of a gene of interest, the nucleotide(s) at that locus or equivalent thereof in one or both alleles form the genotype of the gene at that locus. A genotype can be homozygous or heterozygous. Accordingly, "genotyping" means determining the genotype, that is, the nucleotide(s) at a particular gene locus. Genotyping can also be done by determining the amino acid variant at a particular position of a protein which can be used to deduce the corresponding nucleotide variant(s).

As used herein, the term *"DPYD* nucleic acid" means a nucleic acid molecule the nucleotide sequence of which is uniquely found in a *DPYD* gene. That is, a *"DPYD* nucleic acid" is either a *DPYD* genomic DNA or mRNA/cDNA, having a naturally existing nucleotide sequence encoding a naturally existing DPD protein (wild-type or mutant form). The sequence of an example of a naturally existing *DPYD* nucleic acid is found in GenBank Accession No. NM_000110 (PRI 24-SEP-2006) (*see* SEQ ID NO:1).

As used herein, the term "DPD protein" means a polypeptide molecule the amino acid sequence of which is found uniquely in a DPD protein. That is, "DPD protein" is a naturally existing DPD protein (wild-type or mutant form). The sequence of a wild-type form of a DPD protein is found in GenBank Accession No. NM_000110 (PRI 24-SEP-2006) (*see* SEQ ID NO:2). Other examples of DPYD nucleic acid and DPD protein sequences can be found in Genbank accession number U09178 (PRI 28-DEC-1994).

The term "locus" refers to a specific position or site in a gene sequence or protein. Thus, there may be one or more contiguous nucleotides in a particular gene locus, or one or more amino acids at a particular locus in a polypeptide. Moreover, "locus" may also be used to refer to a particular position in a gene where one or more nucleotides have been deleted, inserted, or inverted.

As used herein, the terms "polypeptide," "protein," and "peptide" are used interchangeably to refer to an amino acid chain in which the amino acid residues are linked by covalent peptide bonds. The amino acid chain can be of any length of at least two amino acids, including full-length proteins. Unless otherwise specified, the terms "polypeptide," "protein," and "peptide" also encompass various modified forms thereof, including but not limited to glycosylated forms, phosphorylated forms, etc.

The terms "primer", "probe," and "oligonucleotide" are used herein interchangeably to refer to a relatively short nucleic acid fragment or sequence. They can be DNA, RNA, or a hybrid thereof, or chemically modified analog or derivatives thereof. Typically, they are single-stranded. However, they can also be double-stranded having two complementing strands which can be separated apart by denaturation. Normally, they have a length of from about 8 nucleotides to about 200 nucleotides, preferably from about 12 nucleotides to about 100 nucleotides, and more preferably about 18 to about 50 nucleotides. They can be labeled with detectable markers or modified in any conventional manners for various molecular biological applications.

The term "isolated" when used in reference to nucleic acids (e.g., genomic DNAs, cDNAs, mRNAs, or fragments thereof) is intended to mean that a nucleic acid molecule is present in a form that is substantially separated from other naturally occurring nucleic acids that are normally associated with the molecule. Specifically, since a naturally existing chromosome (or a viral equivalent thereof) includes a long nucleic acid sequence, an "isolated nucleic acid" as used herein means a nucleic acid molecule having only a portion of the nucleic acid sequence in the chromosome but not one or more other portions present on the same chromosome. More specifically, an "isolated nucleic acid" typically includes no more than 25 kb naturally occurring nucleic acid sequences which immediately flank the nucleic acid in the naturally existing chromosome (or a viral equivalent thereof). However, it is noted that an "isolated nucleic acid" as used herein is distinct from a clone in a conventional library such as genomic DNA library and cDNA library in that the clone in a library is still in admixture with almost all the other nucleic acids of a chromosome or cell. Thus, an "isolated nucleic acid" as used herein also should be substantially separated from other naturally occurring nucleic acids that are on a different chromosome of the same organism. Specifically, an "isolated nucleic acid" means a composition in which the specified nucleic acid molecule is significantly enriched so as to constitute at least 10% of the total nucleic acids in the composition.

An "isolated nucleic acid" can be a hybrid nucleic acid having the specified nucleic acid molecule covalently linked to one or more nucleic acid molecules that are not the nucleic acids naturally flanking the specified nucleic acid. For example, an isolated nucleic acid can be in a vector. In addition, the specified nucleic acid may have a nucleotide sequence that is identical to a naturally occurring nucleic acid or a modified form or mutein thereof having one or more mutations such as nucleotide substitution, deletion/insertion, inversion, and the like.

An isolated nucleic acid can be prepared from a recombinant host cell (in which the nucleic acids have been recombinantly amplified and/or expressed), or can be a chemically synthesized nucleic acid having a naturally occurring nucleotide sequence or an artificially modified form thereof.

The term "isolated polypeptide" as used herein is defined as a polypeptide molecule that is present in a form other than that found in nature. Thus, an isolated polypeptide can be a non-naturally occurring polypeptide. For example, an "isolated polypeptide" can be a "hybrid polypeptide." An "isolated polypeptide" can also be a polypeptide derived from a naturally occurring polypeptide by additions or deletions or substitutions of amino acids. An isolated polypeptide can also be a "purified polypeptide" which is used herein to mean a composition or preparation in which the specified polypeptide molecule is significantly enriched so as to constitute at least 10% of the total protein content in the composition. A "purified polypeptide" can be obtained from natural or recombinant host cells by standard purification techniques, or by chemically synthesis, as will be apparent to skilled artisans.

The terms "hybrid protein," "hybrid polypeptide," "hybrid peptide," "fusion protein," "fusion polypeptide," and "fusion peptide" are used herein interchangeably to mean a non-naturally occurring polypeptide or isolated polypeptide having a specified polypeptide molecule covalently linked to one or more other polypeptide molecules that do not link to the specified polypeptide in nature. Thus, a "hybrid protein" may be two naturally occurring proteins or fragments thereof linked together by a covalent linkage. A "hybrid protein" may also be a protein formed by covalently linking two artificial polypeptides together. Typically but not necessarily, the two or more polypeptide molecules are linked or "fused" together by a peptide bond forming a single non-branched polypeptide chain.

The term "high stringency hybridization conditions," when used in connection with nucleic acid hybridization, means hybridization conducted overnight at 42 degrees C in a solution containing 50% formamide, 5xSSC (750 mM NaCl, 75 mM sodium citrate), 50 mM sodium phosphate, pH 7.6, 5x Denhardt's solution, 10% dextran sulfate, and 20 microgram/ml denatured and sheared salmon sperm DNA, with hybridization filters washed in 0.1xSSC at about 65°C. The term "moderate stringent hybridization conditions," when used in connection with nucleic acid hybridization, means hybridization conducted overnight at 37 degrees C in a solution containing 50% formamide, 5xSSC (750 mM NaCl, 75 mM sodium citrate), 50 mM sodium phosphate, pH 7.6, 5x Denhardt's solution, 10% dextran sulfate, and 20 microgram/ml denatured and sheared salmon sperm DNA, with hybridization filters washed in 1xSSC at about 50°C. It is noted that many other hybridization methods, solutions and temperatures can be used to achieve comparable stringent hybridization conditions as will be apparent to skilled artisans.

For the purpose of comparing two different nucleic acid or polypeptide sequences, one sequence (test sequence) may be described to be a specific "percentage identical to" another sequence (comparison sequence) in the present disclosure. In this respect, the percentage identity is determined by the algorithm of Karlin and Altschul, Proc. Natl. Acad. Sci. USA, 90:5873-5877 (1993), which is incorporated into various BLAST programs. Specifically, the percentage identity is determined by the "BLAST 2 Sequences" tool, which is available at NCBI's website. *See* Tatusova and Madden, FEMS Microbiol. Lett., 174(2):247-250 (1999). For pairwise DNA-DNA comparison, the BLASTN 2.1.2 program is used with default parameters (Match: 1; Mismatch: -2; Open gap: 5 penalties; extension gap: 2 penalties; gap x_dropoff: 50; expect: 10; and word size: 11, with filter). For pairwise protein-protein sequence comparison, the BLASTP 2.1.2 program is employed using default parameters (Matrix: BLOSUM62; gap open: 11; gap extension: 1; x_dropoff: 15; expect: 10.0; and wordsize: 3, with filter). Percent identity of two sequences is calculated by aligning a test sequence with a comparison sequence using BLAST 2.1.2., determining the number of amino acids or nucleotides in the aligned test sequence that are identical to amino acids or nucleotides in the same position of the comparison sequence, and dividing the number of identical amino acids or nucleotides by the number of amino acids or nucleotides in the comparison sequence. When BLAST 2.1.2 is used to compare two sequences, it aligns the sequences and yields the percent identity over defined, aligned regions. If the two sequences are aligned across their entire length, the percent identity yielded by the BLAST 2.1.1 is the percent identity of the two sequences. If BLAST 2.1.2 does not align the two sequences over their entire length, then the number of identical amino acids or nucleotides in the unaligned regions of the test sequence and comparison sequence is considered to be zero and the percent identity is calculated by adding the number of identical amino acids or nucleotides in the aligned regions and dividing that number by the length of the comparison sequence.

The term "reference sequence" refers to a polynucleotide or polypeptide sequence known in the art, including those disclosed in publicly accessible databases, e.g., GenBank, or a newly identified gene sequence, used simply as a reference with respect to the nucleotide variants provided in the present invention. The nucleotide or amino acid sequence in a reference sequence is contrasted to the alleles disclosed in the present invention having newly discovered nucleotide or amino acid variants. In the instant disclosure, genomic DNA corresponding to *DPYD* can be derived from the sequence in GenBank Accession No. NC_000001, while the nucleotide and amino acid sequences in GenBank Accession No. NM_000110 (PRI 24-SEP-2006) (*see* SEQ ID NOs:1 and 2) are used as the reference sequences for *DPYD* mRNA and DPD protein. Another reference sequence of a *DPYD* mRNA and DPD protein is given in U09178. The human *DPYD* gene and DPD coding sequence is reported in the literature and disclosed in SEQ ID NOs:1 and 2. These sequences are representative of one particular individual in the population of humans. Humans vary from one to another in their gene sequences. These variations are very minimal, sometimes occurring at a frequency of about 1 to 10 nucleotides per gene. Different forms of any particular gene exist within the human population. These different forms are called allelic variants. Allelic variants often do not change the amino acid sequence of the encoded protein; such variants are termed synonymous. Even if they do change the encoded amino acid (non-synonymous), the function of the protein is not typically affected. Such changes are evolutionarily or functionally neutral. When human *DPYD* or DPD is referred to in the present application all allelic variants are intended to be encompassed by the term. The sequence of SEQ ID NOs: 1 and 2 are provided merely as representative examples of a wild-type human sequence. The invention is not limited to this single allelic form of *DPYD* or the DPD protein it encodes.

### 2. Nucleotide and Amino Acid Variants

Thus, in accordance with the present invention, genetic variants have been discovered in the *DPYD* gene. The identified polymorphisms are summarized in Table 1 below.

Thus, in accordance with the present invention, a number of genetic variants have been discovered in genetic tests that analyze the *DPYD* gene in different individuals. The variants detected, includes the following variants K63E nt 288 A>G, V162I nt 585 G>A, Y186C nt 658 A>G, I256M nt 869 T>G, S326P nt 1077 T>C, S392P nt 1275 T>C, T468N nt 1504 C>A, T488I nt 1564 C>T, G539R nt 1716 G>A, R561L nt 1783 G>T, V586A nt 1858 T>C, K616Q nt 1947 A>C, D659G nt 2077 A>G, V732G nt 2296 T>G, R783G nt 2448 C>G, K861R nt 2683 A>G, and L993R nt 3079 T>G (all in reference to GenBank accession number NM_000110).

The amino acid substitutions caused by the nucleotide variants are also identified according to conventional practice. For example, K63E means the amino acid variant at position 63 is glutamate in contrast to lysine in the reference sequence. The standard one letter code for amino acids and nucleotides is used throughout, X indicates a stop codon.

**Table 1: Genetic Variants in the DPYD Gene***

| **SNP Position** | **Amino Acid Variant** |
|---|---|
| 288 A>G | K63E |
| 585 G>A | V162I |
| 658 A>G | Y186C |
| 869 T>G | 1256M |
| 1077 T>C | S326P |
| 1275 T>C | S392P |
| 1504 C>A | T468N |
| 1564 C>T | T488I |
| 1716 G>A | G539R |
| 1783 G>T | R561L |
| 1858 T>C | V586A |
| 1947 A>C | K616Q |
| 2077 A>G | D659G |
| 2296 T>G | V732G |
| 2448 C>G | R783G |
| 2683 A>G | K861R |
| 3079 T>G | L993R |
| *85 T>C | C29R |
| *295dcl TCAT | deletion |
| *IVS5+14g>a | |
| *IVS5-8c>t | |
| 496A>G | M166V |
| IVS8+113c>t | |
| IVS9+36a>g | |
| IVS9-51t>g | |
| IVS10-15t>c | |
| IVS10-28g>t | |
| 1218G>A | M406I |
| 1236G>A | E>E |
| IVS11-106t>a | |
| IVS11-119a>g | |
| 1601G>A | S534N |
| 1627A>G | I543V |
| 1896T>C | F632F |
| IVS15+16g>a | |
| IVS15+75a>g | |
| 2194G>A | V732I |
| IVS18-39g>a | |
| 2846A>T | D949V |
| 3067C>T | P1023S |

| | |
|---|---|
| *In reference to SEQ ID NO: 1 and 2 | |

**Table 2**

| Haplotype | Loci | % of Average |
|---|---|---|
| A | TTGCACATTGGGTAGATGAGGAC | -0.2 |
| B | TTGCACATTGGGTAGGTGAGGAC | 2.7 |
| C | CTGCACATTGGGTAGATGAGGAC | 23.5 |
| D | TTGCACATTGGGTAGATGGGGAC | -11.4 |
| E | CTGCGCATCGGGTAGATGAGGAC | -0.3 |
| F | TTGCACATTGGGTAGATGAGAAC | 5.5 |
| G | TTGCACATCGGGTAGATGAGGAC | 12.4 |
| H | TTGCACATTGGGAAGATGAGGAC | -13.6 |
| I | CTGCACATTGGGTAGATGAGAAC | 2.2 |
| J | TTGCACATTGGGTAGATGGGAAC | |
| K | TTGCGCATCGGGTAGATGAGGAC | 0.4 |
| L | TTGCACATTGGGTAGGTGGGGAC | -2.0 |
| M | TTGCACATTGGGTAGACGAGGAC | 33.4 |
| O | CTGCACATTGGGTAGATGGGGAC | |
| P | TTGCACATTGGGAAGATGGGGAC | |

These haplotypes can be used to predict toxicity to treatment. More specifically, these haplotypes are associated with altered DPD expression levels and can be used according to the methods of the invention, to predict toxicity associated with decreased DPD levels. Determination of these haplotypes can be performed with routine techniques. The haplotype information in combination with specific SNPs in DPYD or other pathway genes (proteins) can be used to predict toxicity to fluoropyrimidne treatment (Seek et al. Clin Can Res 11(16): 5886-92), according to the methods of the invention.

### 3. Isolated Nucleic Acids

Accordingly, the present invention provides an isolated *DPYD* nucleic acid containing at least one of the newly discovered nucleotide variants as summarized in Table 1, or one or more nucleotide variants that will result in the amino acid variants provided in Table 1, *e.g*., 288 A>G and 1564 C>T (in reference to GenBank accession number NM_000110 as defined in SEQ ID NO:1 and 2 below, which is the reference sequences used hereafter). It is noted that The term *"DPYD* nucleic acid" is as defined above and means a naturally existing nucleic acid coding for a wild-type or variant or mutant DPD. The term *"DPYD* nucleic acid" is inclusive and may be in the form of either double-stranded or single-stranded nucleic acids, and a singe strand can be either of the two complementing strands. The isolated *DPYD* nucleic acid can be naturally existing genomic DNA, mRNA or cDNA. In one embodiment, the isolated *DPYD* nucleic acid has a nucleotide sequence according to SEQ ID NO:1 but containing one or more exonic nucleotide variants of Table 1 (*e.g*., 288 A>G and 1564 C>T), or the complement thereof.

In another embodiment, the isolated *DPYD* nucleic acid has a nucleotide sequence that is at least 95%, preferably at least 97% and more preferably at least 99% identical to SEQ ID NO: 1 but contains one or more cxonic nucleotide variants of Table 1 (*e.g.,* 288 A>G and1564 C>T), or one or more nucleotide variants that will result in one or more amino acid variants of Table 1, or the complement thereof.

In yet another embodiment, the isolated *DPYD* nucleic acid has a nucleotide sequence encoding DPD protein having an amino acid sequence according to SEQ 1D NO:2 but contains one or more amino acid variants of Table 1 (*e.g*., K63E and T4881). Isolated *DPYD* nucleic acids having a nucleotide sequence that is the complement of the sequence are also encompassed by the present invention.

In yet another embodiment, the isolated *DPYD* nucleic acid has a nucleotide sequence encoding a DPD protein having an amino acid sequence that is at least 95%, preferably at least 97% and more preferably at least 99% identical to SEQ ID NO:2 but contains one or more amino acid variants of Table 1 (e.g., K63E and T4881), or the complement thereof.

The present invention also provides an isolated nucleic acid, naturally occurring or artificial, having a nucleotide sequence that is at least 95%, preferably at least 97% and more preferably at least 99% identical to SEQ ID NO: 1 except for containing one or more nucleotide variants of Table 1 (*e.g*., 288 A>G and1564 C> T), or the complement thereof.

In another embodiment, the present invention provides an isolated nucleic acid, naturally occurring or artificial, having a nucleotide sequence encoding a DPD protein having an amino acid sequence according to SEQ ID NO:2 but containing one or more amino acid variants of Table 1 (*e.g*., corresponding to 288 A>G and1564 C>T). Isolated nucleic acids having a nucleotide sequence that is the complement of the sequence are also encompassed by the present invention.

In addition, isolated nucleic acids are also provided which have a nucleotide sequence encoding a protein having an amino acid sequence that is at least 95%, preferably at least 97% and more preferably at least 99% identical to SEQ ID NO:2 but containing one or more amino acid variants of Table 1 (e.g., K63E and T4881), or the complement thereof.

Also encompassed are isolated *DPYD* nucleic acids obtainable by:
(a) providing a human genomic library;
(b) screening the genomic library using a probe having a nucleotide sequence according to SEQ ID NO: 1; and
(c) producing a genomic DNA comprising a contiguous span of at least 30 nucleotides of any one of SEQ ID NO: 1, wherein the genomic DNA thus produced contains one or more of the polymorphisms of the present invention in Table 1, such as *e.g*., 288 A>G and1564 C>T.

The present invention also includes isolated *DPYD* nucleic acids obtainable by:
(i) providing a cDNA library using human mRNA from a human tissue, e.g., blood;
(ii) screening the cDNA library using a probe having a nucleotide sequence according to SEQ ID NO: 1; and
(iii) producing a cDNA DNA comprising a contiguous span of at least 30 nucleotides of SEQ ID NOs: 1, wherein the cDNA thus produced contains one or more of the SNPs of the present invention in Table 1, such as *e.g*., 288 A>G and1564 C>T.

The present invention also encompasses an isolated nucleic acid comprising the nucleotide sequence of a region of a *DPYD* genomic DNA or cDNA or mRNA, wherein the region contains one or more nucleotide variants as provided in Table 1 above (*e.g*., 288 A>G and 1564 C>T), or one or more nucleotide variants that will give rise to one or more amino acid variants of Table 1, or the complement thereof. Such regions can be isolated and analyzed to efficiently detect the nucleotide variants of the present invention. Also, such regions can also be isolated and used as probes or primers in detection of the nucleotide variants of the present invention and other uses as will be clear from the descriptions below.

Thus, in one embodiment, the isolated nucleic acid comprises a contiguous span of at least 12, 15, 17, 18, 19, 20, 21, 22, 23, 24, 25, 26, 27, 28, 29, 30, 40, 50, 70 or 100 nucleotide residues of a *DPYD* nucleic acid, the contiguous span containing one or more nucleotide variants of Table 1 (*e.g*., 288 A>G and 1564 C>T), or the complement thereof. In specific embodiments, the isolated nucleic acid are oligonucleotides having a contiguous span of from about 17, 18, 19, 20, 21, 22, 23 or 25 to about 30, 40 or 50, preferably from about 21 to about 30 nucleotide residues, of any *DPYD* nucleic acid, said contiguous span containing one or more nucleotide variants of Table I (*e.g*., 288 A>G and 1564 C>T).

In one embodiment, the isolated nucleic acid comprises a contiguous span of at least 12, 15, 17, 18, 19, 20, 21, 22, 23, 24, 25, 26, 27, 28, 29, 30, 40, 50, 70 or 100 nucleotide residues of any one of SEQ ID NO: 1, containing one or more nucleotide variants of Table 1 (e.g., 288 A>G and1564 C>T), or the complement thereof. In specific embodiments, the isolated nucleic acid comprises a nucleotide sequence according to SEQ ID NO: 1. In preferred embodiments, the isolated nucleic acid are oligonucleotides having a contiguous span of from about 17, 18, 19, 20, 21, 22, 23 or 25 to about 30, 40 or 50, preferably from about 21 to about 30 nucleotide residues, of any one of SEQ ID NO:1 and containing one or more nucleotide variants of Table 1 (*e.g*., 288 A>G and1564 C>T). The complements of the isolated nucleic acids are also encompassed by the present invention.

In preferred embodiments, an isolated oligonucleotide of the present invention is specific to a *DPYD* allele ("allele-specific") containing one or more nucleotide variants as disclosed in the present invention. That is, the isolated oligonucleotide is capable of selectively hybridizing, under high stringency conditions generally recognized in the art, to a *DPYD* genomic or cDNA or mRNA containing one or more nucleotide variants as disclosed in the present invention, but not to a *DPYD* gene having a reference sequence of SEQ ID NO: 1. Such oligonucleotides will be useful in a hybridization-based method for detecting the nucleotide variants of the present invention as described in details below. An ordinarily skilled artisan would recognize various stringent conditions which enable the oligonucleotides of the present invention to differentiate between a *DPYD* gene having a reference sequence and a variant *DPYD* gene of the present invention. For example, the hybridization can be conducted overnight in a solution containing 50% formamide, 5xSSC, pH7.6, 5x Denhardt's solution, 10% dextran sulfate, and 20 microgram/ml denatured, sheared salmon sperm DNA. The hybridization filters can be washed in 0.1xSSC at about 65°C. Alternatively, typical PCR conditions employed in the art with an annealing temperature of about 55°C can also be used.

In the isolated *DPYD* oligonucleotides containing a nucleotide variant according to the present invention, the nucleotide variant can be located in any position. In one embodiment, a nucleotide variant is at the 5' or 3' end of the oligonucleotides. In a more preferred embodiment, a *DPYD* oligonucleotide contains only one nucleotide variant according to the present invention, which is located at the 3' end of the oligonucleotide. In another embodiment, a nucleotide variant of the present invention is located within no greater than four (4), preferably no greater than three (3), and more preferably no greater than two (2) nucleotides of the center of the oligonucleotide of the present invention. In more preferred embodiment, a nucleotide variant is located at the center or within one (1) nucleotide of the center of the oligonucleotide. For purposes of defining the location of a nucleotide variant in an oligonucleotide, the center nucleotide of an oligonucleotide with an odd number of nucleotides is considered to be the center. For an oligonucleotide with an even number of nucleotides, the bond between the two center nucleotides is considered to be the center.

In other embodiments of the present invention, isolated nucleic acids are provided which encode a contiguous span of at least 6, 7, 8, 9, 10, 11, 12, 13, 14, 15, 16 or 17 amino acids of a DPD protein wherein said contiguous span contains at least one amino acid variant in Table 1 according to the present invention.

The oligonucleotides of the present invention can have a detectable marker selected from, e.g., radioisotopes, fluorescent compounds, enzymes, or enzyme co-factors operably linked to the oligonucleotide. The oligonucleotides of the present invention can be useful in genotyping as will be apparent from the description below.

In addition, the present invention also provides DNA microchips or microarray incorporating a variant *DPYD* genomic DNA or cDNA or mRNA or an oligonucleotide according to the present invention. The microchip will allow rapid genotyping and/or haplotyping in a large scale.

As is known in the art, in microchips, a large number of different nucleic acid probes are attached or immobilized in an array on a solid support, e.g., a silicon chip or glass slide. Target nucleic acid sequences to be analyzed can be contacted with the immobilized oligonucleotides probes on the microchip. *See* Lipshutz et al., Biotechniques, 19:442-447 (1995); Chee et al., Science, 274:610-614 (1996); Kozal et al., Nat. Med. 2:753-759 (1996); Hacia et al., Nat. Genet., 14:441-447 (1996); Saiki et al., Proc. Natl. Acad. Sci. USA, 86:6230-6234 (1989); Gingeras et al., Genome Res., 8:435-448 (1998). The microchip technologies combined with computerized analysis tools allow large-scale high throughput screening. *See, e.g*., U.S. Patient No. 5,925,525 to Fodor et al; Wilgenbus et al., J. Mol. Med., 77:761-786 (1999); Graber et al., Curr. Opin. Biotechnol., 9:14-18 (1998); Hacia et al., Nat. Genet., 14:441-447 (1996); Shoemaker et al., Nat. Genet., 14:450-456 (1996); DeRisi et al., Nat. Genet., 14:457-460 (1996); Chee et al., Nat. Genet., 14:610-614 (1996); Lockhart et al., Nat. Genet., 14:675-680 (1996); Drobyshev et al., Gene, 188:45-52 (1997).

In a preferred embodiment, a DNA microchip is provided comprising a plurality of the oligonucleotides of the present invention such that the nucleotide identity at each of the nucleotide variant sites disclosed in Table 1 can be determined in one single microarray. In a preferred embodiment, the microchip incorporates variant *DPYD* nucleic acid or oligonucleotide of the present invention and contains at least two of the variants in Table 1, preferably at least three, more preferably at least four of the variants in Table 1.

### 4. DPD Proteins and Peptides

The present invention also provides isolated proteins encoded by one of the isolated nucleic acids according to the present invention. In one aspect, the present invention provides an isolated DPD protein encoded by one of the novel *DPYD* gene variants according to the present invention. Thus, for example, the present invention provides an isolated DPD protein having an amino acid sequence according to SEQ ID NO:2 but containing one or more amino acid variants selected from the group consisting of those disclosed in Table 1. In another example, the isolated DPD protein of the present invention has an amino acid sequence at least 95%, preferably 9%%, more preferably 99% identical to SEQ ID NO:2 wherein the amino acid sequence contains at least one amino acid variant selected from the group consisting of those disclosed in Table 1.

In addition, the present invention also encompasses isolated peptides having a contiguous span of at least 6, 7, 8, 9, 10, 11, 12, 13, 15, 17, 19 or 21 or more amino acids of an isolated DPD protein of the present invention said contiguous span encompassing one or more amino acid variants selected from the group consisting of those disclosed in Table 1. In preferred embodiments, the isolated variant DPD peptides contain no greater than 200 or 100 amino acids, and preferably no greater than 50 amino acids. In specific embodiments, the DPD polypeptides in accordance with the present invention contain one or more of the amino acid variants identified in accordance with the present invention. The peptides can be useful in preparing antibodies specific to the mutant DPD proteins provided in accordance with the present invention.

Thus, as an example, an isolated polypeptide of the present invention can have a contiguous span of at least 6, 7, 8, 9, 10, 11, 12, 13, 14 or 15 amino acid residues of SEQ ID NO:2 encompassing the amino acid variant K63E (amino acid residue No. 63 in SEQ ID NO:2), or a contiguous span of at least 6, 7, 8, 9, 10, 11, 12, 13, 14 or 15 amino acid residues of SEQ ID NO:2 encompassing the amino acid variant T4881 (amino acid residue No. 488 in SEQ ID NO:2).

As will be apparent to an ordinarily skilled artisan, the isolated nucleic acids and isolated polypeptides of the present invention can be prepared using technique generally known in the field of molecular biology. *See generally*, Sambrook et al., Molecular Cloning: A Laboratory Manual, 2nd ed., Cold Spring Harbor Laboratory, Cold Spring Harbor, NY, 1989. The isolated *DPYD* gene or cDNA or oligonucleotides of this invention can be operably linked to one or more other DNA fragments. For example, the isolated *DPYD* nucleic acid (e.g., cDNA or oligonucleotides) can be ligated to another DNA such that a fusion protein can be encoded by the ligation product. The isolated DPYD nucleic acid (e.g., cDNA or oligonucleotides) can also be incorporated into a DNA vector for purposes of, e.g., amplifying the nucleic acid or a portion thereof, and/or expressing a mutant DPD polypeptide or a fusion protein thereof.

Thus, the present invention also provides a vector construct containing an isolated nucleic acid of the present invention, such as a mutant *DPYD* nucleic acid (e.g., cDNA or oligonucleotides) of the present invention. Generally, the vector construct may include a promoter operably linked to a DNA of interest (including a full-length sequence or a fragment thereof in the 5' to 3' direction or in the reverse direction for purposes of producing antisense nucleic acids), an origin of DNA replication for the replication of the vector in host cells and a replication origin for the amplification of the vector in, e.g., *E. coli*, and selection marker(s) for selecting and maintaining only those host cells harboring the vector. Additionally, the vector preferably also contains inducible elements, which function to control the expression of the isolated gene sequence. Other regulatory sequences such as transcriptional termination sequences and translation regulation sequences (e.g., Shine-Dalgarno sequence) can also be included. An epitope tag-coding sequence for detection and/or purification of the encoded polypeptide can also be incorporated into the vector construct. Examples of useful epitope tags include, but are not limited to, influenza virus hemagglutinin (HA), Simian Virus 5 (V5), polyhistidine (6xHis), *c-myc*, lacZ, GST, and the like. Proteins with polyhistidine tags can be easily detected and/or purified with Ni affinity columns, while specific antibodies to many epitope tags are generally commercially available. The vector construct can be introduced into the host cells or organisms by any techniques known in the art, e.g., by direct DNA transformation, microinjection, electroporation, viral infection, lipofection, gene gun, and the like. The vector construct can be maintained in host cells in an extrachromosomal state, *i.e*., as self-replicating plasmids or viruses. Alternatively, the vector construct can be integrated into chromosomes of the host cells by conventional techniques such as selection of stable cell lines or site-specific recombination. The vector construct can be designed to be suitable for expression in various host cells, including but not limited to bacteria, yeast cells, plant cells, insect cells, and mammalian and human cells. A skilled artisan will recognize that the designs of the vectors can vary with the host cell used.

### 5. Antibodies

The present invention also provides antibodies selectively immunoreactive with a variant DPD protein or peptide provided in accordance with the present invention and methods for making the antibodies. As used herein, the term "antibody" encompasses both monoclonal and polyclonal antibodies that fall within any antibody classes, e.g., IgG, IgM, IgA, etc. The term "antibody'' also means antibody fragments including, but not limited to, Fab and F(ab')₂, conjugates of such fragments, and single-chain antibodies that can be made in accordance with U.S. Patent No. 4,704,692, which is incorporated herein by reference. Specifically, the phrase "selectively immunoreactive with one or more of the newly discovered variant DPD protein variants" as used herein means that the immunoreactivity of an antibody with a protein variant of the present invention is substantially higher than that with the DPD protein heretofore known in the art such that the binding of the antibody to the protein variant of the present invention is readily distinguishable, based on the strength of the binding affinities, from the binding of the antibody to the DPD protein having a reference amino acid sequence. Preferably, the binding constant differs by a magnitude of at least 2 fold, more preferably at least 5 fold, even more preferably at least 10 fold, and most preferably at least 100 fold.

To make such an antibody, a variant DPD protein or a peptide of the present invention having a particular amino acid variant (*e.g*., substitution or insertion or deletion) is provided and used to immunize an animal. The variant DPD protein or peptide variant can be made by any methods known in the art, *e.g*., by recombinant expression or chemical synthesis. To increase the specificity of the antibody, a shorter peptide containing an amino acid variant is preferably generated and used as antigen. Techniques for immunizing animals for the purpose of making polyclonal antibodies are generally known in the art. *See* Harlow and Lane, Antibodies: A Laboratory Manual, Cold Spring Harbor Laboratory, Cold Spring Harbor, NY, 1988. A carrier may be necessary to increase the immunogenicity of the polypetide. Suitable carriers known in the art include, but are not limited to, liposome, macromolecular protein or polysaccharide, or combination thereof. Preferably, the carrier has a molecular weight in the range of about 10,000 to 1,000,000. The polypeptide may also be administered along with an adjuvant, *e.g*., complete Freund's adjuvant.

The antibodies of the present invention preferably are monoclonal. Such monoclonal antibodies may be developed using any conventional techniques known in the art. For example, the popular hybridoma method disclosed in Kohler and Milstein, Nature, 256:495-497 (1975) is now a well-developed technique that can be used in the present invention. *See* U.S. Patent No. 4,376,110, which is incorporated herein by reference. Essentially, B-lymphocytes producing a polyclonal antibody against a protein variant of the present invention can be fused with myeloma cells to generate a library of hybridoma clones. The hybridoma population is then screened for antigen binding specificity and also for immunoglobulin class (isotype). In this manner, pure hybridoma clones producing specific homogenous antibodies can be selected. *See generally*, Harlow and Lane, Antibodies: A Laboratory Manual, Cold Spring Harbor Press, 1988. Alternatively, other techniques known in the art may also be used to prepare monoclonal antibodies, which include but are not limited to the EBV hybridoma technique, the human N-cell hybridoma technique, and the trioma technique.

In addition, antibodies selectively immunoreactive with a protein or peptide variant of the present invention may also be recombinantly produced. For example, cDNAs prepared by PCR amplification from activated B-lymphocytes or hybridomas may be cloned into an expression vector to form a cDNA library, which is then introduced into a host cell for recombinant expression. The cDNA encoding a specific protein may then be isolated from the library. The isolated cDNA can be introduced into a suitable host cell for the expression of the protein. Thus, recombinant techniques can be used to recombinantly produce specific native antibodies, hybrid antibodies capable of simultaneous reaction with more than one antigen, chimeric antibodies (e.g., the constant and variable regions are derived from different sources), univalent antibodies which comprise one heavy and light chain pair coupled with the Fc region of a third (heavy) chain, Fab proteins, and the like. *See* U.S. Patent No. 4,816,567; European Patent Publication No. 0088994; Munro, Nature, 312:597 (1984); Morrison, Science, 229:1202 (1985); Oi et al., BioTechniques, 4:214 (1986); and Wood et al., Nature, 314:446-449 (1985), all of which are incorporated herein by reference. Antibody fragments such as Fv fragments, single-chain Fv fragments (scFv), Fab' fragments, and F(ab')₂ fragments can also be recombinantly produced by methods disclosed in, *e.g*., U.S. Patent No. 4,946,778; Skerra & Plückthun, Science, 244:1038-1041(1988); Better et al., Science, 240:1041-1043 (1988); and Bird, et al., Science, 242:423-426 (1988), all of which are incorporated herein by reference.

In a preferred embodiment, the antibodies provided in accordance with the present invention are partially or fully humanized antibodies. For this purpose, any methods known in the art may be used. For example, partially humanized chimeric antibodies having V regions derived from the tumor-specific mouse monoclonal antibody, but human C regions are disclosed in Morrison and Oi, Adv. Immunol., 44:65-92 (1989). In addition, fully humanized antibodies can be made using transgenic non-human animals. For example, transgenic non-human animals such as transgenic mice can be produced in which endogenous immunoglobulin genes are suppressed or deleted, while heterologous antibodies are encoded entirely by exogenous immunoglobulin genes, preferably human immunoglobulin genes, recombinantly introduced into the genome. *See e.g*., U.S. Patent Nos. 5,530,101; 5,545,806; 6,075,181; PCT Publication No. WO 94/02602; Green et. al., Nat. Genetics, 7: 13-21 (1994); and Lonberg et al., Nature 368: 856-859 (1994), all of which are incorporated herein by reference. The transgenic non-human host animal may be immunized with suitable antigens such as a protein variant of the present invention to illicit specific immune response thus producing humanized antibodies. In addition, cell lines producing specific humanized antibodies can also be derived from the immunized transgenic non-human animals. For example, mature B-lymphocytes obtained from a transgenic animal producing humanized antibodies can be fused to myeloma cells and the resulting hybridoma clones may be selected for specific humanized antibodies with desired binding specificities. Alternatively, cDNAs may be extracted from mature B-lymphocytes and used in establishing a library which is subsequently screened for clones encoding humanized antibodies with desired binding specificities.

In a specific embodiment, the antibody is selectively immunoreactive with a variant DPD protein or peptide containing one or more of the amino acid variants disclosed in Table 1.

### 6. Genotyping

The present invention also provides a method for genotyping the *DPYD* gene by determining whether an individual has a nucleotide variant or amino acid variant of the present invention.

Similarly, a method for haplotyping the *DPYD* gene is also provided. Haplotyping can be done by any methods known in the art. For example, only one copy of the *DPYD* gene can be isolated from an individual and the nucleotide at each of the variant positions is determined. Alternatively, an allele specific PCR or a similar method can be used to amplify only one copy of the *DPYD* gene in an individual, and the SNPs at the variant positions of the present invention are determined. The Clark method known in the art can also be employed for haplotyping. A high throughput molecular haplotyping method is also disclosed in Tost et al., Nucleic Acids Res., 30(19):e96(2002), which is incorporated herein by reference.

Thus, additional variant(s) that are in linkage disequilibrium with the variants and/or haplotypes of the present invention can be identified by a haplotyping method known in the art, as will be apparent to a skilled artisan in the field of genetics and haplotying. The additional variants that are in linkage disequilibrium with a variant or haplotype of the present invention can also be useful in the various applications as described below.

For purposes of genotyping and haplotyping, both genomic DNA and mRNA/cDNA can be used, and both are herein referred to generically as "gene."

Numerous techniques for detecting nucleotide variants are known in the art and can all be used for the method of this invention. The techniques can be protein-based or DNA-based. In either case, the techniques used must be sufficiently sensitive so as to accurately detect the small nucleotide or amino acid variations. Very often, a probe is utilized which is labeled with a detectable marker. Unless otherwise specified in a particular technique described below, any suitable marker known in the art can be used, including but not limited to, radioactive isotopes, fluorescent compounds, biotin which is detectable using strepavidin, enzymes (*e.g*., alkaline phosphatase), substrates of an enzyme, ligands and antibodies, etc. *See* Jablonski et al., Nucleic Acids Res., 14:6115-6128 (1986); Nguyen et al., Biotechniques, 13: 116-123 (1992); Rigby et al., J. Mol. Biol., 113:237-251 (1977).

In a DNA-based detection method, target DNA sample, *i.e*., a sample containing *DPYD* genomic DNA or cDNA or mRNA must be obtained from the individual to be tested. Any tissue or cell sample containing the *DPYD* genomic DNA, mRNA, or cDNA or a portion thereof can be used. For this purpose, a tissue sample containing cell nucleus and thus genomic DNA can be obtained from the individual. Blood samples can also be useful except that only white blood cells and other lymphocytes have cell nucleus, while red blood cells are anucleus and contain only mRNA. Nevertheless, mRNA is also useful as it can be analyzed for the presence of nucleotide variants in its sequence or serve as template for cDNA synthesis. The tissue or cell samples can be analyzed directly without much processing. Alternatively, nucleic acids including the target sequence can be extracted, purified, and/or amplified before they are subject to the various detecting procedures discussed below. Other than tissue or cell samples, cDNAs or genomic DNAs from a cDNA or genomic DNA library constructed using a tissue or cell sample obtained from the individual to be tested are also useful.

To determine the presence or absence of a particular nucleotide variant, one technique is simply sequencing the target genomic DNA or cDNA, particularly the region encompassing the nucleotide variant locus to be detected. Various sequencing techniques are generally known and widely used in the art including the Sanger method and Gilbert chemical method. The newly developed pyrosequencing method monitors DNA synthesis in real time using a luminometric detection system. Pyrosequencing has been shown to be effective in analyzing genetic polymorphisms such as single-nucleotide polymorphisms and thus can also be used in the present invention. *See* Nordstrom et al., Biotechnol. Appl. Biochem., 31(2):107-112 (2000); Ahmadian et al., Anal. Biochem., 280:103-110(2000).

Alternatively, the restriction fragment length polymorphism (RFLP) and AFLP method may also prove to be useful techniques. In particular, if a nucleotide variant in the target *DPYD* DNA results in the elimination or creation of a restriction enzyme recognition site, then digestion of the target DNA with that particular restriction enzyme will generate an altered restriction fragment length pattern. Thus, a detected RFLP or AFLP will indicate the presence of a particular nucleotide variant.

Another useful approach is the single-stranded conformation polymorphism assay (SSCA), which is based on the altered mobility of a single-stranded target DNA spanning the nucleotides variant of interest. A single nucleotide change in the target sequence can result in different intramolecular base pairing pattern, and thus different secondary structure of the single-stranded DNA, which can be detected in a non-denaturing gel. *See* Orita et al., Proc. Natl. Acad. Sci. USA, 86:2776-2770 (1989). Denaturing gel-based techniques such as clamped denaturing gel electrophoresis (CDGE) and denaturing gradient gel electrophoresis (DGGE) detect differences in migration rates of mutant sequences as compared to wild-type sequences in denaturing gel. *See* Miller et al., Biotechniques, 5:1016-24 (1999); Sheffield et al., Am. J. Hum, Genet., 49:699-706 (1991); Wartell et al., Nucleic Acids Res., 18:2699-2705 (1990); and Sheffield et al., Proc. Natl. Acad. Sci. USA, 86:232-236 (1989). In addition, the double-strand conformation analysis (DSCA) can also be useful in the present invention. *See* Arguello et al., Nat. Genet., 18:192-194 (1998).

The presence or absence of a nucleotide variant at a particular locus in the *DPYD* gene of an individual can also be detected using the amplification refractory mutation system (ARMS) technique. *See e.g*., European Patent No. 0,332,435; Newton et al., Nucleic Acids Res., 17:2503-2515 (1989); Fox et al., Br. J. Cancer, 77:1267-1274 (1998); Robertson et al., Eur. Respir. J., 12:477-482 (1998). In the ARMS method, a primer is synthesized matching the nucleotide sequence immediately 5' upstream from the locus being tested except that the 3'-end nucleotide which corresponds to the nucleotide at the locus is a predetermined nucleotide. For example, the 3'-end nucleotide can be the same as that in the mutated locus. The primer can be of any suitable length so long as it hybridizes to the target DNA under stringent conditions only when its 3'-end nucleotide matches the nucleotide at the locus being tested. Preferably the primer has at least 12 nucleotides, more preferably from about 18 to 50 nucleotides. If the individual tested has a mutation at the locus and the nucleotide therein matches the 3'-end nucleotide of the primer, then the primer can be further extended upon hybridizing to the target DNA template, and the primer can initiate a PCR amplification reaction in conjunction with another suitable PCR primer. In contrast, if the nucleotide at the locus is of wild type, then primer extension cannot be achieved. Various forms of ARMS techniques developed in the past few years can be used. *See e.g*., Gibson et al., Clin. Chem. 43:1336-1341 (1997).

Similar to the ARMS technique is the mini sequencing or single nucleotide primer extension method, which is based on the incorporation of a single nucleotide. An oligonucleotide primer matching the nucleotide sequence immediately 5' to the locus being tested is hybridized to the target DNA or mRNA in the presence of labeled dideoxyribonucleotides. A labeled nucleotide is incorporated or linked to the primer only when the dideoxyribonucleotides matches the nucleotide at the variant locus being detected. Thus, the identity of the nucleotide at the variant locus can be revealed based on the detection label attached to the incorporated dideoxyribonucleotides. *See* Syvanen et al., Genomics, 8:684-692 (1990); Shumaker et al., Hum, Mutat, 7:346-354 (1996); Chen et al., Genome Res., 10:549-547 (2000).

Another set of techniques useful in the present invention is the so-called "oligonucleotide ligation assay" (OLA) in which differentiation between a wild-type locus and a mutation is based on the ability of two oligonucleotides to anneal adjacent to each other on the target DNA molecule allowing the two oligonucleotides joined together by a DNA ligase. *See* Landergren et al., Science, 241.1077-1080 (1988); Chen et al, Genome Res., 8 :549-556 (1998); Iannone et al., Cytometry, 39:131-140 (2000). Thus, for example, to detect a single-nucleotide mutation at a particular locus in the *DPYD* gene, two oligonucleotides can be synthesized, one having the *DPYD* sequence just 5' upstream from the locus with its 3' end nucleotide being identical to the nucleotide in the variant locus of the *DPYD* gene, the other having a nucleotide sequence matching the *DPYD* sequence immediately 3' downstream from the locus in the *DPYD* gene. The oligonucleotides can be labeled for the purpose of detection. Upon hybridizing to the target *DPYD* gene under a stringent condition, the two oligonucleotides are subject to ligation in the presence of a suitable ligase. The ligation of the two oligonucleotides would indicate that the target DNA has a nucleotide variant at the locus being detected.

Detection of small genetic variations can also be accomplished by a variety of hybridization-based approaches. Allele-specific oligonucleotides are most useful. *See* Conner et al., Proc. Natl. Acad. Sci. USA, 80:278-282 (1983); Saiki et al, Proc. Natl. Acad. Sci. USA, 86:6230-6234 (1989). Oligonucleotide probes (allele-specific) hybridizing specifically to a *DPYD* gene allele having a particular gene variant at a particular locus but not to other alleles can be designed by methods known in the art. The probes can have a length of, *e.g*., from 10 to about 50 nucleotide bases. The target *DPYD* DNA and the oligonucleotide probe can be contacted with each other under conditions sufficiently stringent such that the nucleotide variant can be distinguished from the wild-type *DPYD* gene based on the presence or absence of hybridization. The probe can be labeled to provide detection signals. Alternatively, the allele-specific oligonucleotide probe can be used as a PCR amplification primer in an "allele-specific PCR" and the presence or absence of a PCR product of the expected length would indicate the presence or absence of a particular nucleotide variant.

Other useful hybridization-based techniques allow two single-stranded nucleic acids annealed together even in the presence of mismatch due to nucleotide substitution, insertion or deletion. The mismatch can then be detected using various techniques. For example, the annealed duplexes can be subject to electrophoresis. The mismatched duplexes can be detected based on their electrophoretic mobility that is different from the perfectly matched duplexes. *See* Cariello, Human Genetics, 42:726 (1988). Alternatively, in a RNase protection assay, a RNA probe can be prepared spanning the nucleotide variant site to be detected and having a detection marker. *See* Giunta et al., Diagn. Mol. Path., 5:265-270 (1996); Finkelstein et al., Genomics, 7:167-172 (1990); Kinszler et al., Science 251:1366-1370 (1991). The RNA probe can be hybridized to the target DNA or mRNA forming a heteroduplex that is then subject to the ribonuclease RNase A digestion. RNase A digests the RNA probe in the heteroduplex only at the site of mismatch. The digestion can be determined on a denaturing electrophoresis gel based on size variations. In addition, mismatches can also be detected by chemical cleavage methods known in the art. *See e.g*., Roberts et al., Nucleic Acids Res., 25:3377-3378 (1997).

In the mutS assay, a probe can be prepared matching the *DPYD* gene sequence surrounding the locus at which the presence or absence of a mutation is to be detected, except that a predetermined nucleotide is used at the variant locus. Upon annealing the probe to the target DNA to form a duplex, the *E. coli* mutS protein is contacted with the duplex. Since the mutS protein binds only to heteroduplex sequences containing a nucleotide mismatch, the binding of the mutS protein will be indicative of the presence of a mutation. See Modrich et al., Ann. Rev. Genet., 25:229-25 3 (1991).

A great variety of improvements and variations have been developed in the art on the basis of the above-described basic techniques, and can all be useful in detecting mutations or nucleotide variants in the present invention. For example, the "sunrise probes" or "molecular beacons" utilize the fluorescence resonance energy transfer (FRET) property and give rise to high sensitivity. *See* Wolf et al., Proc. Nat. Acad. Sci. USA, 85:8790-8794 (1988). Typically, a probe spanning the nucleotide locus to be detected are designed into a hairpin-shaped structure and labeled with a quenching fluorophore at one end and a reporter fluorophore at the other end. In its natural state, the fluorescence from the reporter fluorophore is quenched by the quenching fluorophore due to the proximity of one fluorophore to the other. Upon hybridization of the probe to the target DNA, the 5' end is separated apart from the 3'-end and thus fluorescence signal is regenerated. See Nazarenko et al., Nucleic Acids Res., 25:2516-2521 (1997); Rychlik et al., Nucleic Acids Res., 17:8543-8551 (1989); Sharkey et al., Bio/Technology 12:506-509 (1994); Tyagi et al., Nat. Biotechnol., 14:303-308 (1996); Tyagi et al., Nat. Biotechnol., 16:49-53 (1998). The homo-tag assisted non-dimer system (HANDS) can be used in combination with the molecular beacon methods to suppress primer-dimer accumulation. *See* Brownie et al., Nucleic Acids Res., 25:3235-3241 (1997).

Dye-labeled oligonucleotide ligation assay is a FRET-based method, which combines the OLA assay and PCR. *See* Chen et al., Genome Res. 8:549-556 (1998). TaqMan is another FRET-based method for detecting nucleotide variants. A TaqMan probe can be oligonucleotides designed to have the nucleotide sequence of the *DPYD* gene spanning the variant locus of interest and to differentially hybridize with different *DPYD* alleles. The two ends of the probe are labeled with a quenching fluorophore and a reporter fluorophore, respectively. The TaqMan probe is incorporated into a PCR reaction for the amplification of a target *DPYD* gene region containing the locus of interest using Taq polymerase. As Taq polymerase exhibits 5'-3' exonuclease activity but has no 3'-5' exonuclease activity, if the TaqMan probe is annealed to the target *DPYD* DNA template, the 5'-end of the TaqMan probe will be degraded by Taq polymerase during the PCR reaction thus separating the reporting fluorophore from the quenching fluorophore and releasing fluorescence signals. *See* Holland et al., Proc. Natl. Acad. Sci. USA, 88:7276-7280 (1991). Kalinina et al., Nucleic Acids Res., 25:1999-2004 (1997); Whitcombe et al., Clin. Chem., 44:918-923 (1998).

In addition, the detection in the present invention can also employ a chemiluminescence-based technique. For example, an oligonucleotide probe can be designed to hybridize to either the wild-type or a variant *DPYD* gene locus but not both. The probe is labeled with a highly chemiluminescent acridinium ester. Hydrolysis of the acridinium ester destroys chemiluminescence. The hybridization of the probe to the target DNA prevents the hydrolysis of the acridinium ester. Therefore, the presence or absence of a particular mutation in the target DNA is determined by measuring chemiluminescence changes. *See* Nelson et al., Nucleic Acids Res., 24:4998-5003 (1996).

The detection of genetic variation in the *DPYD* gene in accordance with the present invention can also be based on the "base excision sequence scanning" (BESS) technique. The BESS method is a PCR-based mutation scanning method. BESS T-Scan and BESS G-Tracker are generated which are analogous to T and G ladders of dideoxy sequencing. Mutations are detected by comparing the sequence of normal and mutant DNA. *See*, *e.g*., Hawkins et al., Electrophoresis, 20:1171-1176 (1999).

Another useful technique that is gaining increased popularity is mass spectrometry. See Graber et al., Curr. Opin. Biotechnol., 9:14-18 (1998). For example, in the primer oligo base extension (PROBE^{™}) method, a target nucleic acid is immobilized to a solid-phase support. A primer is annealed to the target immediately 5' upstream from the locus to be analyzed. Primer extension is carried out in the presence of a selected mixture of deoxyribonucelotides and dideoxyribonucleotides. The resulting mixture of newly extended primers is then analyzed by MALDI-TOF. *See e.g*., Monforte et al., Nat. Med., 3:360-362 (1997).

In addition, the microchip or microarray technologies are also applicable to the detection method of the present invention. Essentially, in microchips, a large number of different oligonucleotide probes are immobilized in an array on a substrate or carrier, e.g., a silicon chip or glass slide. Target nucleic acid sequences to be analyzed can be contacted with the immobilized oligonucleotide probes on the microchip. *See* Lipshutz et al., Biotechniques, 19:442-447 (1995); Chee et al., Science, 274:610-614 (1996); Kozal et al., Nat. Med. 2:753-759 (1996); Hacia et al., Nat. Genet., 14:441-447 (1996); Saiki et al., Proc. Natl. Acad. Sci. USA, 86:6230-6234 (1989); Gingeras et al., Genome Res., 8:435-448 (1998). Alternatively, the multiple target nucleic acid sequences to be studied are fixed onto a substrate and an array of probes is contacted with the immobilized target sequences. *See* Drmanac et al., Nat. Biotechnol., 16:54-58 (1998). Numerous microchip technologies have been developed incorporating one or more of the above described techniques for detecting mutations. The microchip technologies combined with computerized analysis tools allow fast screening in a large scale. The adaptation of the microchip technologies to the present invention will be apparent to a person of skill in the art apprised of the present disclosure. *See*, *e.g*., U.S. Patent No. 5,925,525 to Fodor et al; Wilgenbus et al., J. Mol. Med., 77:761-786 (1999); Graber *et al*., *Curr. Opin. Biotechnol*., 9:14-18 (1998); Hacia et al., Nat. Genet., 14:441-447 (1996); Shoemaker et al., Nat. Genet., 14:450-456 (1996); DeRisi et al., Nat. Genet., 14:457-460 (1996); Chee et al., Nat. Genet., 14:610-614 (1996); Lockhart et al., Nat. Genet., 14:675-680 (1996); Drobyshev et al., Gene, 188:45-52 (1997).

As is apparent from the above survey of the suitable detection techniques, it may or may not be necessary to amplify the target DNA, *i.e*., the *DPYD* gene or cDNA or mRNAto increase the number of target DNA molecule, depending on the detection techniques used. For example, most PCR-based techniques combine the amplification of a portion of the target and the detection of the mutations. PCR amplification is well known in the art and is disclosed in U.S. Patent Nos. 4,683,195 and 4,800,159, both which are incorporated herein by reference. For non-PCR-based detection techniques, if necessary, the amplification can be achieved by, e.g., *in vivo* plasmid multiplication, or by purifying the target DNA from a large amount of tissue or cell samples. *See generally*, Sambrook et al., Molecular Cloning: A Laboratory Manual, 2nd ed., Cold Spring Harbor Laboratory, Cold Spring Harbor, NY, 1989. However, even with scarce samples, many sensitive techniques have been developed in which small genetic variations such as single-nucleotide substitutions can be detected without having to amplify the target DNA in the sample. For example, techniques have been developed that amplify the signal as opposed to the target DNA by, *e.g*., employing branched DNA or dendrimers that can hybridize to the target DNA. The branched or dendrimer DNAs provide multiple hybridization sites for hybridization probes to attach thereto thus amplifying the detection signals. *See* Detmer et al., J. Clin. Microbiol., 34:901-907 (1996); Collins et al., Nucleic Acids Res., 25:2979-2984 (1997); Horn et al., Nucleic Acids Res., 25:4835-4841 (1997); Horn et al., Nucleic Acids Res., 25:4842-4849 (1997); Nilsen et al., J. Theor. Biol., 187:273-284 (1997).

In yet another technique for detecting single nucleotide variations, the Invader^{®} assay utilizes a novel linear signal amplification technology that improves upon the long turnaround times required of the typical PCR DNA sequenced-based analysis. *See* Cooksey et al., Antimicrobial Agents and Chemotherapy 44:1296-1301 (2000). This assay is based on cleavage of a unique secondary structure formed between two overlapping oligonucleotides that hybridize to the target sequence of interest to form a "flap." Each "flap" then generates thousands of signals per hour. Thus, the results of this technique can be easily read, and the methods do not require exponential amplification of the DNA target. The Invader^{®} system utilizes two short DNA probes, which are hybridized to a DNA target. The structure formed by the hybridization event is recognized by a special cleavase enzyme that cuts one of the probes to release a short DNA "flap." Each released "flap" then binds to a fluorescently-labeled probe to form another cleavage structure. When the cleavase enzyme cuts the labeled probe, the probe emits a detectable fluorescence signal. *See e.g.* Lyamichev et al., Nat. Biotechnol., 17:292-296 (1999).

The rolling circle method is another method that avoids exponential amplification. Lizardi et al., Nature Genetics, 19:225-232 (1998) (which is incorporated herein by reference). For example, Sniper™, a commercial embodiment of this method, is a sensitive, high-throughput SNP scoring system designed for the accurate fluorescent detection of specific variants. For each nucleotide variant, two linear, allele-specific probes are designed. The two allele-specific probes are identical with the exception of the 3'-base, which is varied to complement the variant site. In the first stage of the assay, target DNA is denatured and then hybridized with a pair of single, allele-specific, opencircle oligonucleotide probes. When the 3'-base exactly complements the target DNA, ligation of the probe will preferentially occur. Subsequent detection of the circularized oligonucleotide probes is by rolling circle amplification, whereupon the amplified probe products are detected by fluorescence. *See* Clark and Pickering, Life Science News 6, 2000, Amersham Pharmacia Biotech (2000).

A number of other techniques that avoid amplification all together include, e.g., surface-enhanced resonance Raman scattering (SERRS), fluorescence correlation spectroscopy, and single-molecule electrophoresis. In SERRS, a chromophore-nucleic acid conjugate is absorbed onto colloidal silver and is irradiated with laser light at a resonant frequency of the chromophore. *See* Graham et al., Anal. Chem., 69:4703-4707 (1997). The fluorescence correlation spectroscopy is based on the spatio-temporal correlations among fluctuating light signals and trapping single molecules in an electric field. *See* Eigen et al., Proc. Natl. Acad. Sci. USA, 91:3740-5747 (1994). In single-molecule electrophoresis, the electrophoretic velocity of a fluorescently tagged nucleic acid is determined by measuring the time required for the molecule to travel a predetermined distance between two laser beams. *See* Castro et al., Anal. Chem., 67:3181-3186(1995).

In addition, the allele-specific oligonueleotides (ASO) can also be used in *in situ* hybridization using tissues or cells as samples. The oligonucleotide probes which can hybridize differentially with the wild-type gene sequence or the gene sequence harboring a mutation may be labeled with radioactive isotopes, fluorescence, or other detectable markers. *In situ* hybridization techniques are well known in the art and their adaptation to the present invention for detecting the presence or absence of a nucleotide variant in the *DPYD* gene of a particular individual should be apparent to a skilled artisan apprised of this disclosure.

Protein-based detection techniques may also prove to be useful, especially when the nucleotide variant causes amino acid substitutions or deletions or insertions or frameshift that affect the protein primary, secondary or tertiary structure. To detect the amino acid variations, protein sequencing techniques may be used. For example, a DPD protein or fragment thereof can be synthesized by recombinant expression using an *DPYD* DNA fragment isolated from an individual to be tested. Preferably, a DPD cDNA fragment of no more than 100 to 150 base pairs encompassing the polymorphic locus to be determined is used. The amino acid sequence of the peptide can then be determined by conventional protein sequencing methods. Alternatively, the recently developed HPLC-microscopy tandem mass spectrometry technique can be used for determining the amino acid sequence variations. In this technique, proteolytic digestion is performed on a protein, and the resulting peptide mixture is separated by reversed-phase chromatographic separation. Tandem mass spectrometry is then performed and the data collected therefrom is analyzed. *See* Gatlin et al., Anal. Chem., 72:757-763 (2000).

Other useful protein-based detection techniques include immunoaffinity assays based on antibodies selectively immunoreactive with mutant DPD proteins according to the present invention. The method for producing such antibodies is described above in detail. Antibodies can be used to immunoprecipitate specific proteins from solution samples or to immunoblot proteins separated by, e.g., polyacrylamide gels. Immunocytochemical methods can also be used in detecting specific protein polymorphisms in tissues or cells. Other well-known antibody-based techniques can also be used including, e.g., enzyme-linked immunosorbent assay (ELISA), radioimmunoassay (RIA), immunoradiometric assays (IRMA) and immunoenzymatic assays (IEMA), including sandwich assays using monoclonal or polyclonal antibodies. *See e.g*., U.S. Patent Nos. 4,376,110 and 4,486,530, both of which are incorporated herein by reference.

Accordingly, the presence or absence of a *DPYD* nucleotide variant or amino acid variant in an individual can be determined using any of the detection methods described above.

Typically, once the presence or absence of a *DPYD* nucleotide variant or an amino acid variant resulting from a nucleotide variant of the present invention is determined, physicians or genetic counselors or patients or other researchers may be informed of the result. Specifically the result can be cast in a transmittable form that can be communicated or transmitted to other researchers or physicians or genetic counselors or patients. Such a form can vary and can be tangible or intangible. The result with regard to the presence or absence of a *DPYD* nucleotide variant of the present invention in the individual tested can be embodied in descriptive statements, diagrams, photographs, charts, images or any other visual forms. For example, images of gel electrophoresis of PCR products can be used in explaining the results. Diagrams showing where a variant occurs in an individual's *DPYD* gene are also useful in indicating the testing results. The statements and visual forms can be recorded on a tangible media such as papers, computer readable media such as floppy disks, compact disks, *etc*., or on an intangible media, e.g., an electronic media in the form of email or website on internet or intranet. In addition, the result with regard to the presence or absence of a nucleotide variant or amino acid variant of the present invention in the individual tested can also be recorded in a sound form and transmitted through any suitable media, *e.g*., analog or digital cable lines, fiber optic cables, etc., via telephone, facsimile, wireless mobile phone, internet phone and the like.

Thus, the information and data on a test result can be produced anywhere in the world and transmitted to a different location. For example, when a genotyping assay is conducted offshore, the information and data on a test result may be generated and cast in a transmittable form as described above. The test result in a transmittable form thus can be imported into the U.S. Accordingly, the present invention also encompasses a method for producing a transmittable form of information on the *DPYD* genotype of an individual. The method comprises the steps of (1) determining the presence or absence of a nucleotide variant according to the present invention in the *DPYD* gene of the individual; and (2) embodying the result of the determining step in a transmittable form. The transmittable form is the product of the production method.

The present invention also provides a kit for genotyping *DPYD* gene, *i.e*., determining the presence or absence of one or more of the nucleotide or amino acid variants of present invention in a *DPYD* gene in a sample obtained from a patient. The kit may include a carrier for the various components of the kit. The carrier can be a container or support, in the form of, *e.g*., bag, box, tube, rack, and is optionally compartmentalized. The carrier may define an enclosed confinement for safety purposes during shipment and storage. The kit also includes various components useful in detecting nucleotide or amino acid variants discovered in accordance with the present invention using the above-discussed detection techniques.

In one embodiment, the detection kit includes one or more oligonucleotides useful in detecting one or more of the nucleotide variants in *DPYD* gene. Preferably, the oligonucleotides are allele-specific, *i.e*., are designed such that they hybridize only to a mutant *DPYD* gene containing a particular nucleotide variant discovered in accordance with the present invention, under stringent conditions. Thus, the oligonucleotides can be used in mutation-detecting techniques such as allele-specific oligonucleotides (ASO), allele-specific PCR, TaqMan, chemiluminescence-based techniques, molecular beacons, and improvements or derivatives thereof, e.g., microchip technologies. The oligonucleotides in this embodiment preferably have a nucleotide sequence that matches a nucleotide sequence of a variant *DPYD* gene allele containing a nucleotide variant to be detected. The length of the oligonucleotides in accordance with this embodiment of the invention can vary depending on its nucleotide sequence and the hybridization conditions employed in the detection procedure. Preferably, the oligonucleotides contain from about 10 nucleotides to about 100 nucleotides, more preferably from about 15 to about 75 nucleotides, e.g., contiguous span of 18, 19, 20, 21, 22, 23, 24 or 25 to 21, 22, 23, 24, 26, 27, 28, 29 or 30 nucleotide residues of a *DPYD* nucleic acid one or more of the residues being a nucleotide variant of the present invention, *i.e*., selected from Table 1. Under most conditions, a length of 18 to 30 may be optimum. In any event, the oligonucleotides should be designed such that it can be used in distinguishing one nucleotide variant from another at a particular locus under predetermined stringent hybridization conditions. Preferably, a nucleotide variant is located at the center or within one (1) nucleotide of the center of the oligonucleotides, or at the 3' or 5' end of the oligonucleotides. The hybridization of an oligonucleotide with a nucleic acid and the optimization of the length and hybridization conditions should be apparent to a person of skill in the art. *See generally,* Sambrook et al., Molecular Cloning: A Laboratory Manual, 2nd ed., Cold Spring Harbor Laboratory, Cold Spring Harbor, NY, 1989. Notably, the oligonucleotides in accordance with this embodiment are also useful in mismatch-based detection techniques described above, such as electrophoretic mobility shift assay, RNase protection assay, mutS assay, etc.

In another embodiment of this invention, the kit includes one or more oligonucleotides suitable for use in detecting techniques such as ARMS, oligonucicotide ligation assay (OLA), and the like. The oligonucleotides in this embodiment include a *DPYD* gene sequence of about 10 to about 100 nucleotides, preferably from about 15 to about 75 nucleotides, *e.g*., contiguous span of 18, 19, 20, 21, 22, 23, 24 or 25 to 21, 22, 23, 24, 26, 27, 28, 29 or 30 nucleotide residues immediately 5' upstream from the nucleotide variant to be analyzed. The 3' end nucleotide in such oligonucleotides is a nucleotide variant in accordance with this invention.

The oligonucleotides in the detection kit can be labeled with any suitable detection marker including but not limited to, radioactive isotopes, fluorephores, biotin, enzymes (e.g., alkaline phosphatase), enzyme substrates, ligands and antibodies, etc. *See* Jablonski et al., Nucleic Acids Res., 14:6115-6128 (1986); Nguyen et al., Biotechniques, 13:116-123 (1992); Rigby et al., J. Mol. Biol., 113:237-251 (1977). Alternatively, the oligonucleotides included in the kit are not labeled, and instead, one or more markers are provided in the kit so that users may label the oligonucleotides at the time of use.

In another embodiment of the invention, the detection kit contains one or more antibodies selectively immunoreactive with certain DPD proteins or polypeptides containing specific amino acid variants discovered in the present invention. Methods for producing and using such antibodies have been described above in detail.

Various other components useful in the detection techniques may also be included in the detection kit of this invention. Examples of such components include, but are not limited to, Taq polymerase, deoxyribonucleotides, dideoxyribonucleotides other primers suitable for the amplification of a target DNA sequence, RNase A, mutS protein, and the like. In addition, the detection kit preferably includes instructions on using the kit for detecting nucleotide variants in *DPYD* gene sequences.

### 7. Use of Genotyping in Diagnosis Applications

The present invention further relates to methods of determining in an individual an increased likelihood of toxicity from a drug that is metabolized by DPD. The genotyping methods can also be used to determine in an individual an increased likelihood of response to a drug that is metabolized by DPD. As indicated above, the present invention provides *DPYD* polymorphisms associated with susceptibility to toxicity to drug metabolized by the DPD enzyme, *e.g*., 5-FU. Specifically, the polymorphism 288 A>G and 1564 C>T and those in Table 1 are believed to be associated with susceptibility to toxicity to drug metabolized by the DPD enzyme, *e.g*., 5-FU.

Thus, in one aspect, the present invention encompasses a method for predicting or detecting an increased susceptibility toxicity to a drug metabolized by DPD (*e.g*., a fluoropyrimidine like 5-FU or capecitabine) in an individual, which comprises the step of genotyping the individual to determine the individual's genotype at one or more of the loci identified in the present invention, or another locus at which the genotype is in linkage disequilibrium with one of the polymorphisms of the present invention. Thus, if one or more of the polymorphism, *e.g*., 288 A>G and 1564 C>T, is detected then it can be reasonably predicted that the individual is at an increased risk of having an adverse reaction to therapeutic treatment with 5-FU (or capecitabine). In particular, if an individual is homozygous with the genotype 288 A>G or 1564 C>T, then it can be reasonably predicted that the individual has a higher susceptibility to toxicity to drugs metabolized by the DPD enzyme, *e.g*., 5-FU. If the individual has a biallelic deleterious mutation wherein one allele has either 288 A>G or 1564 C>T, and the other allele has a known DPD deficiency causing alteration, then the individual is likely to have a higher susceptibility to toxicity to drugs metabolized by the DPD enzyme, e.g., 5-FU. In other words, such an individual has an increased likelihood or is at an increased risk toxicity to 5-FU. If an individual is heterozygous for one or more of the genetic variants (e.g., 288 A>G or 1564 C>T) then his or her risk of having a toxic reaction to 5-FU is intermediate. On the other hand, if one or more of the genetic variants of the present invention (e.g., 288 A>G or 1564 C>T) is not detected in the individual, and does not have other variants associated with 5-FU toxicity, then it can be reasonably predicted that the individual has a low susceptibility of toxicity to drugs metabolized by DPD, particularly 5-FU and capecitabine.

### 8. Cell and Anima Models

In yet another aspect of the present invention, a cell line and a transgenic animal carrying a *DPYD* gene containing one or more of the nucleotide variants in accordance with the present invention are provided. The cell line and transgenic animal can be used as a model system for studying cancers and testing various therapeutic approaches in treating cancers.

To establish the cell line, cells expressing the variant DPD protein can be isolated from an individual canning the nucleotide variants. The primary cells can be transformed or immortalized using techniques known in the art. Alternatively, normal cells expressing a wild-type DPD protein or other type of nucleotide variants can be manipulated to replace the entire endogenous *DPYD* gene with a variant *DPYD* gene containing one or more of the nucleotide variants in accordance with the present invention, or simply to introduce mutations into the endogenous *DPYD* gene. The genetically engineered cells can further be immortalized.

A more valuable model system is a transgenic animal. A transgenic animal can be made by replacing the endogenous animal *DPYD* gene with a variant DPD gene containing one or more of the nucleotide variants in accordance with the present invention. Alternatively, insertions and/or deletions can be introduced into the endogenous animal *DPYD* gene to simulate the *DPYD* alleles discovered in accordance with the present invention. Techniques for making such transgenic animals are well known and are described in, *e.g.*, Capecchi, et al., Science, 244:12288 (1989); Hasty et al., Nature, 350:243 (1991); Shinkai et al., Cell, 68:855 (1992); Mombaerts et al., Cell, 68:869 (1992); Philpott et at., Science, 256:1448 (1992); Snouwaert et al., Science, 257:1083 (1992); Donehower et al., Nature, 356:215 (1992); Hogan et al., Manipulating the Mouse Embryo; A Laboratory Manual, 2nd edition, Cold Spring Harbor Laboratory Press, 1994; and U.S. Patent Nos. 5,800,998, 5,891,628, and 4,873,191, all of which are incorporated herein by reference.

The cell line and transgenic animal are valuable tools for studying the variant DPD genes, and in particular for testing *in vivo* the compounds identified in the screening method of this invention and other therapeutic approaches as discussed below. As is known in the art, studying drug candidates in a suitable animal model before advancing them into human clinical trials is particularly important because efficacy of the drug candidates can be confirmed in the model animal, and the toxicology profiles, side effects, and dosage ranges can be determined. Such information is then used to guide human clinical trials.

### 9. Therapeutic Applications

As discussed above, the DPD protein variants provided in accordance with the present invention are likely to be defective in activities in metabolism of 5-FU and other drugs metabolized by the enzyme. Thus, once an individual is identified as having one of such variants and is determined to have a DPD deficiency (as determined by the methods provided in the present invention), the individual can be plated under prophylactic or therapeutic treatment.

In one embodiment, a normal or wild-type DPD protein may be administered directly to the patient. For this purpose, the normal or wild-type DPD protein may be prepared by any one of the methods described in Section 4 may be administered to the patient, preferably in a pharmaceutical composition as described below. Proteins isolated or purified from normal individuals or recombinantly produced can all be used in this respect.

In another embodiment, gene therapy approaches are employed to supply functional DPD proteins to a patient in need of treatment. For example, a nucleic acid encoding a normal or wild-type DPD protein may be introduced into tissue cells of a patient such that the protein is expressed from the introduced nucleic acids. The exogenous nucleic acid can be used to replace the corresponding endogenous defective gene by, *e.g.*, homologous recombination. *See* U.S. Patent No. 6,010,908, which is incorporated herein by reference. Alternatively, if the disease-causing mutation is a recessive mutation, the exogenous nucleic acid is simply used to express a wild-type protein in addition to the endogenous mutant protein. In another approach, the method disclosed in U.S. Patent No. 6,077,705 may be employed in gene therapy. That is, the patient is administered both a nucleic acid construct encoding a ribozyme and a nucleic acid construct comprising a ribozymc resistant gene encoding a wild type form of the gene product. As a result, undesirable expression of the endogenous gene is inhibited and a desirable wild-type exogenous gene is introduced.

Various gene therapy methods are well known in the art. Successes in gene therapy have been reported recently. *See e.g.,* Kay et al., Nature Genet., 24:257-61 (2000); Cavazzana-Calvo et al., Science, 288:669 (2000); and Blaese et al., Science, 270: 475 (1995); Kantoff, et al., J. Exp. Med. 166:219 (1987).

Any suitable gene therapy methods can be used for purposes of the present invention. Generally, a nucleic acid encoding a desirable functional DPD protein is incorporated into a suitable expression vector and is operably plinked to a promoter in the vector. Suitable promoters include but are not limited to viral transcription promoters derived from adenovirus, simian virus 40 (SV40) (*e.g.*, the early and late promoters of SV40), Rous sarcoma virus (RSV), and cytomegalovirus (CMV) (*e.g.*, CMV immediate-early promoter), human immunodeficiency virus (HIV) (*e.g.*, long terminal repeat (LTR)), vaccinia virus (*e.g.*, 7.5K promoter), and herpes simplex virus (HSV) (*e.g.*, thymidine kinase promoter). Where tissue-specific expression of the exogenous gene is desirable, tissue-specific promoters may be operably linked to the exogenous gene. In addition, selection markers may also be included in the vector for purposes of selecting, *in vitro*, those cells that contain the exogenous gene. Various selection markers known in the art may be used including, but not limited to, *e.g.*, genes conferring resistance to neomycin, hygromycin, zeocin, and the like.

In one embodiment, the exogenous nucleic acid (gene) is incorporated into a plasmid DNA vector. Many commercially available expression vectors may be useful for the present invention, including, *e.g.*, pCEP4, pcDNAI, pIND, pSecTag2, pVAX1, pcDNA3.1, and pBI-ECFP, and pDisplay.

Various viral vectors may also be used. Typically, in a viral vector, the viral genome is engineered to eliminate the disease-causing capability, *e.g.*, the ability to replicate in the host cells. The exogenous nucleic acid to be introduced into a patient may be incorporated into the engineered viral genome, *e.g.*, by inserting it into a viral gene that is non-essential to the viral infectivity. Viral vectors are convenient to use as they can be easily introduced into tissue cells by way of infection. Once in the host cell, the recombinant virus typically is integrated into the genome of the host cell. In rare instances, the recombinant virus may also replicate and remain as extrachromosomal elements.

A large number of retroviral vectors have been developed for gene therapy. These include vectors derived from oncoretroviruses (*e.g.*, MLV), lentiviruses (*e.g.*, HIV and SIV) and other retroviruses. For example, gene therapy vectors have been developed based on murine leukemia virus (*See*, Cepko, et al., Cell, 37:1053-1062 (1984), Cone and Mulligan, Proc. Natl. Acad. Sci. U.S.A., 81:6349-6353 (1984)), mouse mammary tumor virus (*See*, Salmons et al., Biochem, Biophys. Res. Commun., 159:1191-1198 (1984)), gibbon ape leuheraia virus (See, Miller et al., J. Virology, 65:2220-2224 (1991)), HIV, (*See* Shimada et al., J. Clin . Invest., 88:1043-1047 (1991)), and avian retroviruses (*See* Cosset et al., J. Virology, 64: 1070-1078 (1990)). In addition, various retroviral vectors are also described in U.S. Patent Nos. 6,168,916; 6,140,111; 6,096,534; 5,985,655: 5,911,983; 4,980,286; and 4,868,116, all of which are incorporated herein by reference.

Adeno-associated virus (AAV) vectors have been successfully tested in clinical trials. *See e.g.,* Kay et al., Nature Genet. 24:257-61 (2000). AAV is a naturally occurring defective virus that requires other viruses such as adenoviruses or herpes viruses as helper viruses. *See* Muzyczka, Curr. Top. Microbiol. Immun., 158:97 (1992).

A recombinant AAV virus useful as a gene therapy vector is disclosed in U.S. Patent No. 6,153,436, which is incorporated herein by reference.

Adenoviral vectors can also be useful for purposes of gene therapy in accordance with the present invention. For example, U.S. Patent No. 6,001,816 discloses an adenoviral vector, which is used to deliver a leptin gene intravenously to a mammal to treat obesity. Other recombinant adenoviral vectors may also be used, which include those disclosed in U.S. Patent Nos. 6,171,855; 6,140,087; 6,063,622; 6,033,908; and 5,932,210, and Rosenfeld et al., Science, 252:431-434 (1991); and Rosenfeld et al., Cell, 68: 143-155 (1992):

Other useful viral vectors include recombinant hepatitis viral vectors (*See*, *e.g.*, U.S. Patent No. 5,981,274), and recombinant entomopox vectors (*See*, *e.g.*, U.S. Patent Nos. 5,721,352 and 5,753,258).

Other non-traditional vectors may also be used for purposes of this invention. For example, International Publication No. WO 94/18834 discloses a method of delivering DNA into mammalian cells by conjugating the DNA to be delivered with a polyelectrolyte to form a complex. The complex may be microinjected into or uptaken by cells.

The exogenous gene fragment or plasmid DNA vector containing the exogenous gene may also be introduced into cells by way of receptor-mediated endocytosis. *See e.g.*, U.S. Patent No. 6,090,619; Wu and Wu, J. Biol. Chem., 263:14621 (1988); Curiel et al., Proc. Natl. Acad. Sci. USA, 88:8850 (1991). For example, U.S. Patent No. 6,083,741 discloses introducing an exogenous nucleic acid into mammalian cells by associating the nucleic acid to a polycation moiety (*e.g.*, poly-L-lysine having 3-100 lysine residues), which is itself coupled to an integrin receptor binding moiety (*e.g.*, a cyclic peptide having the sequence RGD).

Alternatively, the exogenous nucleic acid or vectors containing it can also be delivered into cells via amphiphiles. *See e.g.*, U.S. Patent No. 6,071,890. Typically, the exogenous nucleic acid or a vector containing the nucleic acid forms a complex with the cationic amphiphile. Mammalian cells contacted with the complex can readily take the complex up.

The exogenous gene can be introduced into a patient for purposes of gene therapy by various methods known in the art. For example, the exogenous gene sequences alone or in a conjugated or complex form described above, or incorporated into viral or DNA vectors, may be administered directly by injection into an appropriate tissue or organ of a patient. Alternatively, catheters or like devices may be used for delivery into a target organ or tissue. Suitable catheters are disclosed in, *e.g.*, U.S. Patent Nos. 4,186,745; 5,397,307; 5,547,472; 5,674,192; and 6,129,705, all of which are incorporated herein by reference.

In addition, the exogenous gene or vectors containing the gene can be introduced into isolated cells using any known techniques such as calcium phosphate precipitation, microinjection, lipofection, electroporation, gene gun, receptor-mediated endocytosis, and the like. Cells expressing the exogenous gene may be selected and redelivered back to the patient by, *e.g.*, injection or cell transplantation. The appropriate amount of cells delivered to a patient will vary with patient conditions, and desired effect, which can be determined by a skilled artisan. *See e.g.*, U.S. Patent Nos. 6,054,288; 6,048,524; and 6,048,729. Preferably, the cells used are autologous, *i.e.*, cells obtained from the patient being treated.

### 10. Pharmaceutical Compositions and Formulations

In another aspect of the present invention, pharmaceutical compositions are also provided containing one or more of the therapeutic agents provided in the present invention. The compositions are prepared as a pharmaceutical formulation suitable for administration into a patient. Accordingly, the present invention also extends to pharmaceutical compositions, medicaments, drugs or other compositions containing one or more of the therapeutic agent in accordance with the present invention.

In the pharmaceutical composition, an active compound identified in accordance with the present invention can be in any pharmaceutically acceptable salt form. As used herein, the term "pharmaceutically acceptable salts" refers to the relatively non-toxic, organic or inorganic salts of the compounds of the present invention, including inorganic or organic acid addition salts of the compound. Examples of such salts include, but are not limited to, hydrochloride salts, sulfate salts, bisulfate salts, borate salts, nitrate salts, acetate salts, phosphate salts, hydrobromide salts, laurylsulfonate salts, glucoheptonate salts, oxalate salts, oleate salts, laurate saltes, stearate salts, palmitate salts, valerate salts, benzoate salts, naththylate salts, mesylate salts, tosylate salts, citrate salts, lactate salts, maleate salts, succinate salts, tartrate salts, fumarate salts, and the like. See, *e.g.*, Berge, et al., J. Pharm. Sci., 66:1-19 (1977).

For oral delivery, the active compounds can be incorporated into a formulation that includes pharmaceutically acceptable carriers such as binders (*e.g.*, gelatin, cellulose, gum tragacanth), excipients (*e.g.*, starch, lactose), lubricants (*e.g.*, magnesium stearate, silicon dioxide), disintegrating agents (*e.g.*, alginate, Primogel, and corn starch), and sweetening or flavoring agents (*e.g.*, glucose, sucrose, saccharin, methyl salicylate, and peppermint). The formulation can be orally delivered in the form of enclosed gelatin capsules or compressed tablets. Capsules and tablets can be prepared in any conventional techniques. The capsules and tablets can also be coated with various coatings known in the art to modify the flavors, tastes, colors, and shapes of the capsules and tablets. In addition, liquid carriers such as fatty oil can also be included in capsules.

Suitable oral formulations can also be in the form of suspension, syrup, chewing gum, wafer, elixir, and the like. If desired, conventional agents for modifying flavors, tastes, colors, and shapes of the special forms can also be included. In addition, for convenient administration by enteral feeding tube in patients unable to swallow, the active compounds can be dissolved in an acceptable lipophilic vegetable oil vehicle such as olive oil, corn oil and safflower oil.

The active compounds can also be administered parenterally in the form of solution or suspension, or in lyophilized form capable of conversion into a solution or suspension form before use. In such formulations, diluents or pharmaceutically acceptable carriers such as sterile water and physiological saline buffer can be used. Other conventional solvents, pH buffers, stabilizers, anti-bacteria agents, surfactants, and antioxidants can all be included. For example, useful components include sodium chloride, acetates, citrates or phosphates buffers, glycerin, dextrose, fixed oils, methyl parabens, polyethylene glycol, propylene glycol, sodium bisulfate, benzyl alcohol, ascorbic acid, and the like. The parenteral formulations can be stored in any conventional containers such as vials and ampoules.

Routes of topical administration include nasal, bucal, mucosal, rectal, or vaginal applications. For topical administration, the active compounds can be formulated into lotions, creams, ointments, gels, powders, pastes, sprays, suspensions, drops and aerosols. Thus, one or more thickening agents, humectants, and stabilizing agents can be included in the formulations. Examples of such agents include, but are not limited to, polyethylene glycol, sorbitol, xanthan gum, petrolatum, beeswax, or mineral oil, lanolin, squalene, and the like. A special form of topical administration is delivery by a transdermal patch. Methods for preparing transdermal patches are disclosed, *e.g.*, in Brown, et al., Annual Review of Medicine, 39:221-229 (1988), which is incorporated herein by reference.

Subcutaneous implantation for sustained release of the active compounds may also be a suitable route of administration. This entails surgical procedures for implanting an active compound in any suitable formulation into a subcutaneous space, *e.g.*, beneath the anterior abdominal wall. *See, e.g.,* Wilson et al., J. Clin. Psych. 45:242-247 (1984). Hydrogels can be used as a carrier for the sustained release of the active compounds. Hydrogels are generally known in the art. They are typically made by cross-linking high molecular weight biocompatible polymers into a network, which swells in water to form a gel like material. Preferably, hydrogels is biodegradable or biosorbable. For purposes of this invention, hydrogels made of polyethylene glycols, collagen, or poly(glycolic-co-L-lactic acid) may be useful. *See, e.g.*, Fhillips et al., J. Pharmaceut. Sci. 73:1718-1720 (1984).

The active compounds can also be conjugated, to a water soluble non-immunogenic non-peptidic high molecular weight polymer to form a polymer conjugate. For example, an active compound is covalently linked to polyethylene glycol to form a conjugate. Typically, such a conjugate exhibits improved solubility, stability, and reduced toxicity and immunogenicity. Thus, when administered to a patient, the active compound in the conjugate can have a longer half life in the body, and exhibit better efficacy. *See generally*, Burnham, Am. J. Hosp. Pharm., 15.210-218 (1994). PEGylated proteins are currently being used in protein replacement therapies and for other therapeutic uses. For example, PEGylated interferon (PEG-INTRON A^{®}) is clinically used for treating Hepatitis B. PEGylated adenosine deaminase (ADAGEN^{®}) is being used to treat severe combined immunodeficiency disease (SCIDS). PEGylated L-asparaginase (ONCAPSPAR^{®}) is being used to threat acute lymphoblastic leukemia (ALL). It is preferred that the covalent linkage between the polymer and the active compound and/or the polymer itself is hydrolytically degradable under physiological conditions. Such conjugates known as "prodrugs" can readily release the active compound inside the body. Controlled release of an active compound can also be achieved by incorporating the active ingredient into microcapsule, nanocapsules, or hydrogels generally known in the art.

Liposomes can also be used as carriers for the active compounds of the present invention. Liposomes are micelles made of various lipids such as cholesterol, phospholipids, fatty acids, and derivatives thereof. Various modified lipids can also be used. Liposomes can reduce the toxicity of the active compounds, and increase their stability. Methods for preparing liposomal suspensions containing active ingredients therein are generally known in the art. *See, e.g.*, U.S. Patent No. 4,522,811; Prescott, Ed., Methods in Cell Biology, Volume XIV, Academic Press, New York, N.Y. (1976).

The active compounds can also be administered in combination with another active agent that synergistically treats or prevents the same symptoms or is effective for another disease or symptom in the patient treated so long as the other active agent does not interfere with or adversely affect the effects of the active compounds of this invention. Such other active agents include but are not limited to anti-inflammation agents, antiviral agents, antibiotics, antifungal agents, antithrombotic agents, cardiovascular drugs, cholesterol lowering agents, hypertension drugs, and other anticancer drugs, and the like.

Generally, the toxicity profile and therapeutic efficacy of the therapeutic agents can be determined by standard pharmaceutical procedures in cell models or animal models, *e.g.*, those provided in Section 7. As is known in the art, the LD₅₀ represents the dose lethal to about 50% of a tested population. The ED₅₀ is a parameter indicating the dose therapeutically effective in about 50% of a tested population. Both LD₅₀ and ED₅₀ can be determined in cell models and animal models. In addition, the IC₅₀ may also be obtained in cell models and animal models, which stands for the circulating plasma concentration that is effective in achieving about 50% of the maximal inhibition of the symptoms of a disease or disorder. Such data may be used in designing a dosage range for clinical trials in humans. Typically, as will be apparent to skilled artisans, the dosage range for human use should be designed such that the range centers around the ED₅₀ and/or IC₅₀, but significantly below the LD₅₀ obtained from cell or animal models.

It will be apparent to skilled artisans that therapeutically effective amount for each active compound to be included in a pharmaceutical composition of the present invention can vary with factors including but not limited to the activity of the compound used, stability of the active compound in the patient's body, the severity of the conditions to be alleviated, the total weight of the patient treated, the route of administration, the ease of absorption, distribution, and excretion of the active compound by the body, the age and sensitivity of the patient to be treated, and the like. The amount of administration can also be adjusted as the various factors change over time.

### Example 1: Identification of DPD Variants

Screening of a population panel identified the novel SNPs in Table 1 that give rise to novel amino acid changes in DPD. All exons and the proximal promoter of the DPYD gene were PCR amplifies using exon-specific primers and PCR products were PCR sequenced by dye-primer chemistry. These variants are believed to be related to deficient DPD activity.

SEQ ID NO:1

1 tttcgactcg cgctccggct gctgtcactt ggctctctgg ctggagcttg aggacgcaag
61 gagggtttgt cactggcaga ctcgagactg taggcactgc catggcccct gtgctcagta
121 aggactcggc ggacatcgag agtatcctgg ctttaaatcc tcgaacacaa actcatgcaa
181 ctctgtgttc cacttcggcc aagaaattag acaagaaaca ttggaaaaga aatcctgata
241 agaactgctt taattgtgag aagctggaga ataattttga tgacatcaag cacacgactc
301 ttggtgagcg aggagctctc cgagaagcaa tgagatgcct gaaatgtgca gatgccccgt
361 gtcagaagag ctgtccaact aatcttgata ttaaatcatt catcacaagt attgcaaaca
421 agaactatta tggagctgct aagatgatat tttctgacaa cccacttggt ctgacttgtg
481 gaatggtatg tccaacctct gatctatgtg taggtggatg caatttatat gccactgaag
541 agggacccat taatattggt ggattgcagc aatttgctac tgaggtattc aaagcaatga
601 gtatcccaca gatcagaaat ccttcgctgc ctcccccaga aaaaatgtct gaagcctatt
661 ctgcaaagat tgctcttttt ggtgctgggc ctgcaagtat aagttgtgct tcctttttgg
721 ctcgattggg gtactctgac atcactatat ttgaaaaaca agaatatgtt ggtggtttaa
781 gtacttctga aattcctcag ttccggctgc cgtatgatgt agtgaatttt gagattgagc

841 taatgaagga ccttggtgta aagataattt gcggtaaaag cctttcagtg aatgaaatga
901 ctcttagcac tttgaaagaa aaaggctaca aagctgcttt cattggaata ggtttgccag
961 aacccaataa agatgccatc ttccaaggcc tgacgcagga ccaggggttt tatacatcca
1021 aagacttttt gccacttgta gccaaaggca gtaaagcagg aatgtgcgcc tgtcactctc
1081 cattgccatc gatacgggga gtcgtgattg tacttggagc tggagacact gccttcgact
1141 gtgcaacatc tgctctacgt tgtggagctc gccgagtgtt catcgtcttc agaaaaggct
1201 ttgttaatat aagagctgtc cctgaggaga tggagcttgc taaggaagaa aagtgtgaat
1261 ttctgccatt cctgtcccca cggaaggtta tagtaaaagg tgggagaatt gttgctatgc
1321 agtttgttcg gacagagcaa gatgaaactg gaaaatggaa tgaagatgaa gatcagatgg
1381 tccatctgaa agccgatgtg gtcatcagtg cctttggttc agttctgagt gatcctaaag
1441 taaaagaagc cttgagccct ataaaattta acagatgggg tctcccagaa gtagatccag
1501 aaactatgca aactagtgaa gcatgggtat ttgcaggtgg tgatgtcgtt ggtttggcta
1561 acactacagt ggaatcggtg aatgatggaa agcaagcttc ttggtacatt cacaaatacg
1621 tacagtcaca atatggagct tccgtttctg ccaagcctga actacccctc ttttacactc
1681 ctattgatct ggtggacatt agtgtagaaa tggccggatt gaagtttata aatccttttg
1741 gtcttgctag cgcaactcca gccaccagca catcaatgat tcgaagagct tttgaagctg
1801 gatggggttt tgccctcacc aaaactttct ctcttgataa ggacattgtg acaaatgttt
1861 cccccagaat catccgggga accacctctg gccccatgta tggccctgga caaagctcct
1921 ttctgaatat tgagctcatc agtgagaaaa cggctgcata ttggtgtcaa agtgtcactg
1981 aactaaaggc tgacttccca gacaacattg tgattgctag cattatgtgc agttacaata
2041 aaaatgactg gacggaactt gccaagaagt ctgaggattc tggagcagat gccctggagt
2101 taaatttatc atgtccacat ggcatgggag aaagaggaat gggcctggcc tgtgggcagg
2161 atccagagct ggtgcggaac atctgccgct gggttaggca agctgttcag attccttttt
2221 ttgccaagct gaccccaaat gtcactgata ttgtgagcat cgcaagagct gcaaaggaag
2281 gtggtgccaa tggcgttaca gccaccaaca ctgtctcagg tctgatggga ttaaaatctg
2341 atggcacacc ttggccagca gtggggattg caaagcgaac tacatatgga ggagtgtctg
2401 ggacagcaat cagacctatt gctttgagag ctgtgacctc cattgctcgt gctctgcctg
2461 gatttcccat tttggctact ggtggaattg actctgctga aagtggtctt cagtttctcc
2521 atagtggtgc ttccgtcctc caggtatgca gtgccattca gaatcaggat ttcactgtga
2581 tcgaagacta ctgcactggc ctcaaagccc tgctttatct gaaaagcatt gaagaactac
2641 aagactggga tggacagagt ccagctactg tgagtcacca gaaagggaaa ccagttccac
2701 gtatagctga actcatggac aagaaactgc caagttttgg accttatctg gaacagcgca
2761 agaaaatcat agcagaaaac aagattagac tgaaagaaca aaatgtagct tttcaccac
2821 ttaagagaag ctgttttatc cccaaaaggc ctattcctac catcaaggat gtaataggaa
2881 aagcactgca gtaccttgga acatttggtg aattgagcaa cgtagagcaa gttgtggcta
2941 tgattgatga agaaatgtgt atcaactgtg gtaaatgcta catgacctgt aatgattctg
3001 gctaccaggc tatacagttt gatccagaaa cccacctgcc caccataacc gacacttgta
3061 caggctgtac tctgtgtctc agtgtttgcc ctattgtcga ctgcatcaaa atggtttcca
3121 ggacaacacc ttatgaacca aagagaggcg tacccttatc tgtgaatccg gtgtgttaag
3181 gtgatttgtg aaacagttgc tgtgaacttt catgtcacct acatatgctg atctcttaaa
3241 atcatgatcc ttgtgttcag ctctttccaa attaaaacaa atatacattt tctaaataaa
3301 aatatgtaat ttcaaaatac atttgtaagt gtaaaaaatg tctcatgtca atgaccattc

3361 aattagtggc ataaaataga ataattcttt tctgaggata gtagttaaat aactgtgtgg
3421 cagttaattg gatgttcact gccagttgtc ttatgtgaaa aattaacttt ttgtgtggca
3481 attagtgtga cagtttccaa attgccctat gctgtgctcc atatttgatt tctaattgta
3541 agtgaaatta agcattttga aacaaagtac tctttaacat acaagaaaat gtatccaagg
3601 aaacatttta tcaataaaaa ttacctttaa ttttaatgct gtttctaaga aaatgtagtt
3661 agctccataa agtacaaatg aagaaagtca aaaattattt gctatggcag gataagaaag
3721 cctaaaattg agtttgtgga ctttattaag taaaatcccc ttcgctgaaa ttgcttattt
3781 ttggtgttgg atagaggata gggagaatat ttactaacta aataccattc actactcatg
3841 cgtgagatgg gtgtacaaac tcatcctctt ttaatggcat ttctctttaa actatgttcc
3901 taaccaaatg agatgatagg atagatcctg gttaccactc ttttactgtg cacatatggg
3961 ccccggaatt ctttaatagt caccttcatg attatagcaa ctaatgtttg aacaaagctc
4021 aaagtatgca atgcttcatt attcaagaat gaaaaatata atgttgataa tatatattaa
4081 gtgtgccaaa tcagtttgac tactctctgt tttagtgttt atgtttaaaa gaaatatatt
4141 ttttgttatt attagataat atttttgtat ttctctattt tcataatcag taaatagtgt
4201 catataaact catttatctc ctcttcatgg catcttcaat atgaatctat aagtagtaaa
4261 tcagaaagta acaatctatg gcttatttct atgacaaatt caagagctag aaaaataaaa
4321 tgtttcatta tgcactttta gaaatgcata tttgccacaa aacctgtatt actgaataat
4381 atcaaataaa atatcataaa gcatttt

SEQ ID NO:2

MAPVLSKDSADIESILALNPRTQTHATLCSTSAKKLDKKHWKRN

PDKNCFNCEKLENNFDDIKHTTLGERGALREAMRCLKCADAPCQKSCPTNLDIKSFIT

SIANKNYYGAAKMIFSDNPLGLTCGMVCPTSDLCVGGCNLYATEEGPINIGGLQQFAT

EVFKAMSIPQIRNPSLPPPEKMSEAYSAKIALFGAGPASISCASFLARLGYSDITIFE

KQEYVGGLSTSEIPQFRLPYDVVNFEIELMKDLGVKIICGKSLSVNEMTLSTLKEKGY

KAAFIGIGLPEPNKDAIFQGLTQDQGFYTSKDFLPLVAKGSKAGMCACHSPLPSIRGV

VIVLGAGDTAFDCATSALRCGARRVFIVFRKGFVNIRAVPEEMELAKEEKCEFLPFLS

PRKVIVKGGRIVAMQFVRTEQDETGKWNEDEDQMVHLKADVVISAFGSVLSDPKVKEA

LSPIKFNRWGLPEVDPETMQTSEAWVFAGGDVVGLANTTVESVNDGKQASWYIHKYVQ

SQYGASVSAKPELPLFYTPIDLVDISVEMAGLKFINPFGLASATPATSTSMIRRAFEA

GWGFALTKTFSLDKDIVTNVSPRIIRGTTSGPMYGPGQSSFLNIELISEKTAAYWCQS

VTELKADFPDNIVIASIMCSYNKNDWTELAKKSEDSGADALELNLSCPHGMGERGMGL

ACGQDPELVRNICRWVRQAVQIPFFAKLTPNVTDIVSIARAAKEGGANGVTATNTVSG

LMGLKSDGTPWPAVGIAKRTTYGGVSGTAIRPIALRAVTSIARALPGFPILATGGIDS

AESGLQFLHSGASVLQVCSAIQNQDFTVIEDYCTGLKALLYLKSIEELQDWDGQSPAT

VSHQKGKPVPRIAELMDKKLPSFGPYLEQRKKIIAENKIRLKEQNVAFSPLKRSCFIP

KRPIPTIKDVIGKALQYLGTFGELSNVEQVVAMIDEEMCINCGKCYMTCNDSGYQAIQ

FDPETHLPTITDTCTGCTLCLSVCPIVDCIKMVSRTTPYEPKRGVPLSVNPVC

### Example 2: DPD activity

DPD Expression in Escherichia coli. For each expression experiment (*e.g.*, the variants disclosed in Table 1), a single colony from a freshly made transformation of DH-5α cells with the expression vector can be inoculated in LB broth and grown to stationary phase. An aliquot from this culture can be used to inoculate 250 ml of terrific broth containing 100 µg/ml ampicillin and supplemented with 100 µM of each FAD and FMN, 100 µM uracil and 10 µM each of Fe(NH₄)₂ (SO₄) and Na₂S. Following a 90 min incubation at 29 C, the trp-lac promoter in the expression vector can be induced by the addition of 1 mM isopropyl-β-d-thiogalacto-pyranoside (IPTG) and the culture can be incubated for an additional 48 h.

The cells can then be sedimented, washed twice with 250 ml of phosphate buffered saline (PBS) and resuspended in 45 ml of 35 mM potassium phosphate buffer (pH 7.3) containing 20% glycerol, 10 mM EDTA, 1 mM. DTT, 0.1 mM PMSF and 2 µM leupeptin. The cell suspension can lysed at 4 C with four 30 sec bursts of a Heat Systems sonicator model W 225-R at 25% of full power (Heat Systems-Ultrasonics, Inc., Plain View N.Y.). The resultant lysate can be centrifuged at 100,000xg for 60 min at 4 C. Solid (NH₄)₂SO₄ can then be slowly added to the supernatant at 4 C with gentle stirring to give a final concentration of 30% saturation. The precipitate can then be sedimented and the pellet containing expressed DPD resuspended in 5 ml of 35 mM potassium phosphate buffer (pH =7.3) containing 1 mM EDTA/1 mM DTT and 0.1 mM PMSF. The protein solution can then be dialyzed at 4 C for 36 h against 3 changes of 4 liters each of buffer and stored at -70 C until further use.

Catalytic assay. DPD activity (*e.g.*, for the variants disclosed in Table 1) can be determined at 37 C by measuring the decrease in absorbance at 340 nm associated with the oxidation of NADPH to NADP⁺. The reaction mixture can contain 28 mM potassium phosphate buffer (pH 7.3), 2 mM MgCl₂, 1 mM DTT, 60 µM NADPH and the expressed DPD in a final volume of 1 ml. The measurements can be carried out using an Aminco DW-2000 double beam spectrophotometer using a blank that contained the complete reaction mixture except substrate. The reactions can be initiated by addition of substrate (uracil, 5-fluorouracil or thymine). The catalytic activity can be calculated as µmole of NADPH oxidized per minute and per mg of expressed DPD. Protein quantities were determined using the bicinchronic (BCA) procedure from Pierce Chemical Co., Rockford, Ill.) following the manufacturer's directions. The skilled artisan is capable of other utilizing other DPD assay systems and modifications to this protocol can be made depending on the application. For example, instead of using recombinant purified DPD, activity can be assessed by using a crude or partially purified preparation obtained from cells of individuals having the particular variant (*e.g.*, for the variants disclosed in Table 1).

All publications and patent applications mentioned in the specification are indicative of the level of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

The present invention also relates to the following items:
1. An isolated nucleic acid or the complement thereof, said isolated nucleic acid comprising a contiguous span of at least 18 nucleotide residues of an *DPYD* nucleic acid, at least one of which being a nucleotide variant selected from the group consisting of those disclosed in Table 1.
2. An isolated oligonucleotide comprising a contiguous span of at least 18 nucleotides of the sequence of a mutant *DPYD* gene, wherein said contiguous span contains a genetic variant selected from the group consisting of those disclosed in Table 1.
3. The isolated oligonucleotide of item 2, wherein said contiguous span contains at least 21 nucleotides of the sequence of said mutant *DPYD* gene.
4. The isolated oligonucleotide of item 3. wherein the genetic variant is at the center of said contiguous span.
5. The isolated oligonucleotide of item 3, wherein the genetic variant is at the 3' end of said oligonucleotide.
6. The isolated oligonucleotide of item 2, wherein said genetic variant is within no more than 3 nucleotides of the center of said contiguous span.
7. The isolated oligonucleotide of item 2, wherein said genetic variant is at the 3' end of said oligonucleotide.
8. The isolated oligonucleotide of item 2, wherein said contiguous span contains at least 50 nucleotides of the sequence of said mutant *DPYD* gene.
9. A DNA microchip comprising the isolated oligonucleotide of item 2.
10. A method for genotyping an individual comprising:
   determining the presence or absence of a genetic variant selected from the group consisting of those disclosed in Table 1 in the *DPYD* gene of the individual.
11. The method of item 10, wherein said determining step comprises sequencing the *DPYD* gene or a portion thereof.
12. A method as in item 10, wherein the presence of said genetic variant is indicative of an increased toxicity to a drug metabolized by the DPD enzyme.
13. A method as in item 12, wherein said drug is 5-FU or capecitabine.
14. A method as in item 12, wherein said drug is 5-FU.
15. The method of item 10, wherein the genetic variant is 288 A>G.
16. The method of item 10, wherein the genetic variant is 585 G>A.
17. The method of item 10, wherein the genetic variant is 658 A>G.
18. The method of item 10, wherein the genetic variant is 869 T>G.
19. The method of item 10, wherein the genetic variant is 1077 T>C.
20. The methods of item 10, wherein the genetic variant is 1275 T>C.
21. The method of item 10, wherein the genetic variant is 1504 C>A.
22. The method of item 10, wherein the genetic variant is 1564 C>T.
23. The method of item 10, wherein the genetic variant is 1716 G>A.
24. The method of item 10, wherein the genetic variant is 1783 G>T.
25. The method of item 10, wherein the genetic variant is 1858 T>C.
26. The method of item 10, wherein the genetic variant is 1947 A>C.
27. The method of item 10, wherein the genetic variant is 2077 A>G.
28. The method of item 10, wherein the genetic variant is 2296 T>G.
29. The method of item 10, wherein the genetic variant is 2448 C>G.
30. The method of item 10, wherein the genetic variant is 2683 A>G.
31. The method of item 10, wherein the genetic variant is 3079 T>G.

## Claims

1. A nucleic acid or the complement thereof, said nucleic acid comprising a contiguous span of at least 18 nucleotide residues of a DPYD nucleic acid, at least one of which being the nucleotide variant 2296 T>G.

2. An oligonucleotide comprising a contiguous span of at least 18 nucleotides of the sequence of a mutant DPYD gene, wherein said contiguous span contains the genetic variant 2296 T>G.

3. The oligonucleotide of claim 2, wherein said contiguous span contains at least 21 nucleotides or at least 50 nucleotides of the sequence of said mutant DPYD gene.

4. The nucleic acid of claim 1 or the oligonucleotides of claim 2 or 3, wherein the genetic variant is at the center or within no more than 3 nucleotides of the center of said contiguous span or at the 3' end of said oligonucleotide.

5. A DNA microchip comprising the oligonucleotide of any one of claims 2 to 4.

6. An in vitro method for genofiyping an individual comprising:
determining whether a DPYD gene of said individual has the genetic variant 2296 T>G.

7. An in vitro method for genofiyping an individual comprising:
determining whether a DPYD gene of said individual has the genetic variant 2296 T>G encoding the amino acid variant V732G.

8. The method of claim 6 or claim 7, wherein said determining step comprises sequencing the DPYD gene or a portion thereof.

9. The method of any one of claims 6 to 8, wherein the presence of said genetic variant is indicative of an increased toxicity to a drug metabolized by the DPD enzyme.

10. The method of claim 9, wherein said drug is 5-FU or capecitabine.


SEQUENCE LISTING

<110> Myriad Genetics, Inc.

<120> DPYD GENE VARIANTS AND USE THEREOF

<130> P3481 EP/1 S3

<150> EP 07 79 9007.5
<151> 2007-06-25

<150> US 60/816,090
<151> 2006-06-23

<150> US 60/863,104
<151> 2006-10-26

<160> 17

<170> PatentIn version 3.4

<210> 1
<211> 4407
<212> DNA
<213> Homo sapiens

<400> 1
tttcgactcg cgctccggct gctgtcactt ggctctctgg ctggagcttg aggacgcaag 60

gagggtttgt cactggcaga ctcgagactg taggcactgc catggcccct gtgctcagta 120

aggactcggc ggacatcgag agtatcctgg ctttaaatcc tcgaacacaa actcatgcaa 180

ctctgtgttc cacttcggcc aagaaattag acaagaaaca ttggaaaaga aatcctgata 240

agaactgctt taattgtgag aagctggaga ataattttga tgacatcaag cacacgactc 300

ttggtgagcg aggagctctc cgagaagcaa tgagatgcct gaaatgtgca gatgccccgt 360

gtcagaagag ctgtccaact aatcttgata ttaaatcatt catcacaagt attgcaaaca 420

agaactatta tggagctgct aagatgatat tttctgacaa cccacttggt ctgacttgtg 480

gaatggtatg tccaacctct gatctatgtg taggtggatg caatttatat gccactgaag 540

agggacccat taatattggt ggattgcagc aatttgctac tgaggtattc aaagcaatga 600

gtatcccaca gatcagaaat ccttcgctgc ctcccccaga aaaaatgtct gaagcctatt 660

ctgcaaagat tgctcttttt ggtgctgggc ctgcaagtat aagttgtgct tcctttttgg 720

ctcgattggg gtactctgac atcactatat ttgaaaaaca agaatatgtt ggtggtttaa 780

gtacttctga aattcctcag ttccggctgc cgtatgatgt agtgaatttt gagattgagc 840

taatgaagga ccttggtgta aagataattt gcggtaaaag cctttcagtg aatgaaatga 900

ctcttagcac tttgaaagaa aaaggctaca aagctgcttt cattggaata ggtttgccag 960

aacccaataa agatgccatc ttccaaggcc tgacgcagga ccaggggttt tatacatcca 1020

aagacttttt gccacttgta gccaaaggca gtaaagcagg aatgtgcgcc tgtcactctc 1080

cattgccatc gatacgggca gtcgtgattg tacttggagc tggagacact gccttcgact 1140

gtgcaacatc tgctctacct tgtggagctc gccgagtgtt catcgtcttc agaaaaggct 1200

ttgttaatat aagagctgtc cctgaggaga tggagcttgc taaggaagaa aagtgtgaat 1260

ttctgccatt cctgtcccca cggaaggtta tagtaaaagg tgggagaatt gttgctatgc 1320

agtttgttcg gacagagcaa gatgaaactg gaaaatggaa tgaagatgaa gatcagatgg 1380

tccatctgaa agccgatgtg gtcatcagtg cctttggttc agttctgagt gatcctaaag 1440

taaaagaagc cttgagccct ataaaattta acagatgggg tctcccagaa gtagatccag 1500

aaactatgca aactagtgaa gcatgggtat ttgcaggtgg tgatgtcgtt ggtttggcta 1560

acactacagt ggaatcggtg aatgatggaa agcaagcttc ttggtacatt cacaaatacg 1620

tacagtcaca atatggagct tccgtttctg ccaagcctga actacccctc ttttacactc 1680

ctattgatct ggtggacatt agtgtagaaa tggccggatt gaagtttata aatccttttg 1740

gtcttgctag cgcaactcca gccaccagca catcaatgat tcgaagagct tttgaagctg 1800

gatggggttt tgccctcacc aaaactttct ctcttgataa ggacattgtg acaaatgttt 1860

cccccagaat catccgggga accacctctg gccccatgta tggccctgga caaagctcct 1920

ttctgaatat tgagctcatc agtgagaaaa cggctgcata ttggtgtcaa agtgtcactg 1980

aactaaaggc tgacttccca gacaacattg tgattgctag cattatgtgc agttacaata 2040

aaaatgactg gacggaactt gccaagaagt ctgaggattc tggagcagat gccctggagt 2100

taaatttatc atgtccacat ggcatgggag aaagaggaat gggcctcgcc tgtgggcagg 2160

atccagagct ggtgcggaac atctgccgct gggttaggca agctgttcag attccttttt 2220

ttgccaagct gaccccaaat gtcactgata ttgtgagcat cgcaagagct gcaaaggaag 2280

gtggtgccaa tggcgttaca gccaccaaca ctgtctcagg tctgatggga ttaaaatctg 2340

atggcacacc ttggccagca gtggggattg caaagcgaac tacatatgga ggagtgtctg 2400

ggacagcaat cagacctatt gctttgagag ctgtgacctc cattgctcgt gctctgcctg 2460

gatttcccat tttggctact ggtggaattg actctgctga aagtggtctt cagtttctcc 2520

atagtggtgc ttccgtcctc caggtatgca gtgccattca gaatcaggat ttcactgtga 2580

tcgaagacta ctgcactggc ctcaaagccc tgctttatct gaaaagcatt gaagaactac 2640

aagactggga tggacagagt ccagctactg tgagtcacca gaaagggaaa ccagttccac 2700

gtatagctga actcatggac aagaaactgc caagttttgg accttatctg gaacagcgca 2760

agaaaatcat agcagaaaac aagattagac tgaaagaaca aaatgtagct ttttcaccac 2820

ttaagagaag ctgttttatc cccaaaaggc ctattcctac catcaaggat gtaataggaa 2880

aagcactgca gtaccttgga acatttggtg aattgagcaa cgtagagcaa gttgtggcta 2940

tgattgatga agaaatgtgt atcaactgtg gtaaatgcta catgacctgt aatgattctg 3000

gctaccaggc tatacagttt gatccagaaa cccacctgcc caccataacc gacacttgta 3060

caggctgtac tctgtgtctc agtgtttgcc ctattgtcga ctgcatcaaa atggtttcca 3120

ggacaacacc ttatgaacca aagagaggcg tacccttatc tgtgaatccg gtgtgttaag 3180

gtgatttgtg aaacagttgc tgtgaacttt catgtcacct acatatgctg atctcttaaa 3240

atcatgatcc ttgtgttcag ctctttccaa attaaaacaa atatacattt tctaaataaa 3300

aatatgtaat ttcaaaatac atttgtaagt gtaaaaaatg tctcatgtca atgaccattc 3360

aattagtggc ataaaataga ataattcttt tctgaggata gtagttaaat aactgtgtgg 3420

cagttaattg gatgttcact gccagttgtc ttatgtgaaa aattaacttt ttgtgtggca 3480

attagtgtga cagtttccaa attgccctat gctgtgctcc atatttgatt tctaattgta 3540

agtgaaatta agcattttga aacaaagtac tctttaacat acaagaaaat gtatccaagg 3600

aaacatttta tcaataaaaa ttacctttaa ttttaatgct gtttctaaga aaatgtagtt 3660

agctccataa agtacaaatg aagaaagtca aaaattattt gctatggcag gataagaaag 3720

cctaaaattg agtttgtgga ctttattaag taaaatcccc ttcgctgaaa ttgcttattt 3780

ttggtgttgg atagaggata gggagaatat ttactaacta aataccattc actactcatg 3840

cgtgagatgg gtgtacaaac tcatcctctt ttaatggcat ttctctttaa actatgttcc 3900

taaccaaatg agatgatagg atagatcctg gttaccactc ttttactgtg cacatatggg 3960

ccccggaatt ctttaatagt caccttcatg attatagcaa ctaatgtttg aacaaagctc 4020

aaagtatgca atgcttcatt attcaagaat gaaaaatata atgttgataa tatatattaa 4080

gtgtgccaaa tcagtttgac tactctctgt tttagtgttt atgtttaaaa gaaatatatt 4140

ttttgttatt attagataat atttttgtat ttctctattt tcataatcag taaatagtgt 4200

catataaact catttatctc ctcttcatgg catcttcaat atgaatctat aagtagtaaa 4260

tcagaaagta acaatctatg gcttatttct atgacaaatt caagagctag aaaaataaaa 4320

tgtttcatta tgcactttta gaaatgcata tttgccacaa aacctgtatt actgaataat 4380

atcaaataaa atatcataaa gcatttt 4407

<210> 2
<211> 1025
<212> PRT
<213> Homo sapiens

<400> 2

Met Ala Pro Val Leu Ser Lys Asp Ser Ala Asp Ile Glu Ser Ile Leu
1               5                   10                  15

Ala Leu Asn Pro Arg Thr Gln Thr His Ala Thr Leu Cys Ser Thr Ser
            20                  25                  30

Ala Lys Lys Leu Asp Lys Lys His Trp Lys Arg Asn Pro Asp Lys Asn
        35                  40                  45

Cys Phe Asn Cys Glu Lys Leu Glu Asn Asn Phe Asp Asp Ile Lys His
    50                  55                  60

Thr Thr Leu Gly Glu Arg Gly Ala Leu Arg Glu Ala Met Arg Cys Leu
65                  70                  75                  80

Lys Cys Ala Asp Ala Pro Cys Gln Lys Ser Cys Pro Thr Asn Leu Asp
                85                  90                  95

Ile Lys Ser Phe Ile Thr Ser Ile Ala Asn Lys Asn Tyr Tyr Gly Ala
            100                 105                 110

Ala Lys Met Ile Phe Ser Asp Asn Pro Leu Gly Leu Thr Cys Gly Met
        115                 120                 125

Val Cys Pro Thr Ser Asp Leu Cys Val Gly Gly Cys Asn Leu Tyr Ala
    130                 135                 140

Thr Glu Glu Gly Pro Ile Asn Ile Gly Gly Leu Gln Gln Phe Ala Thr
145                 150                 155                 160

Glu Val Phe Lys Ala Met Ser Ile Pro Gln Ile Arg Asn Pro Ser Leu
                165                 170                 175

Pro Pro Pro Glu Lys Met Ser Glu Ala Tyr Ser Ala Lys Ile Ala Leu
            180                 185                 190

Phe Gly Ala Gly Pro Ala Ser Ile Ser Cys Ala Ser Phe Leu Ala Arg
        195                 200                 205

Leu Gly Tyr Ser Asp Ile Thr Ile Phe Glu Lys Gln Glu Tyr Val Gly
    210                 215                 220

Gly Leu Ser Thr Ser Glu Ile Pro Gln Phe Arg Leu Pro Tyr Asp Val
225                 230                 235                 240

Val Asn Phe Glu Ile Glu Leu Met Lys Asp Leu Gly Val Lys Ile Ile
                245                 250                 255

Cys Gly Lys Ser Leu Ser Val Asn Glu Met Thr Leu Ser Thr Leu Lys
            260                 265                 270

Glu Lys Gly Tyr Lys Ala Ala Phe Ile Gly Ile Gly Leu Pro Glu Pro

275                 280                 285

Asn Lys Asp Ala Ile Phe Gln Gly Leu Thr Gln Asp Gln Gly Phe Tyr
    290                 295                 300

Thr Ser Lys Asp Phe Leu Pro Leu Val Ala Lys Gly Ser Lys Ala Gly
305                 310                 315                 320

Met Cys Ala Cys His Ser Pro Leu Pro Ser Ile Arg Gly Val Val Ile
                325                 330                 335

Val Leu Gly Ala Gly Asp Thr Ala Phe Asp Cys Ala Thr Ser Ala Leu
            340                 345                 350

Arg Cys Gly Ala Arg Arg Val Phe Ile Val Phe Arg Lys Gly Phe Val
        355                 360                 365

Asn Ile Arg Ala Val Pro Glu Glu Met Glu Leu Ala Lys Glu Glu Lys
    370                 375                 380

Cys Glu Phe Leu Pro Phe Leu Ser Pro Arg Lys Val Ile Val Lys Gly
385                 390                 395                 400

Gly Arg Ile Val Ala Met Gln Phe Val Arg Thr Glu Gln Asp Glu Thr
                405                 410                 415

Gly Lys Trp Asn Glu Asp Glu Asp Gln Met Val His Leu Lys Ala Asp
            420                 425                 430

Val Val Ile Ser Ala Phe Gly Ser Val Leu Ser Asp Pro Lys Val Lys
        435                 440                 445

Glu Ala Leu Ser Pro Ile Lys Phe Asn Arg Trp Gly Leu Pro Glu Val
    450                 455                 460

Asp Pro Glu Thr Met Gln Thr Ser Glu Ala Trp Val Phe Ala Gly Gly
465                 470                 475                 480

Asp Val Val Gly Leu Ala Asn Thr Thr Val Glu Ser Val Asn Asp Gly
                485                 490                 495

Lys Gln Ala Ser Trp Tyr Ile His Lys Tyr Val Gln Ser Gln Tyr Gly
            500                 505                 510

Ala Ser Val Ser Ala Lys Pro Glu Leu Pro Leu Phe Tyr Thr Pro Ile
        515                 520                 525

Asp Leu Val Asp Ile Ser Val Glu Met Ala Gly Leu Lys Phe Ile Asn
    530                 535                 540

Pro Phe Gly Leu Ala Ser Ala Thr Pro Ala Thr Ser Thr Ser Met Ile
545                 550                 555                 560

Arg Arg Ala Phe Glu Ala Gly Trp Gly Phe Ala Leu Thr Lys Thr Phe
                565                 570                 575

Ser Leu Asp Lys Asp Ile Val Thr Asn Val Ser Pro Arg Ile Ile Arg
            580                 585                 590

Gly Thr Thr Ser Gly Pro Met Tyr Gly Pro Gly Gln Ser Ser Phe Leu
        595                 600                 605

Asn Ile Glu Leu Ile Ser Glu Lys Thr Ala Ala Tyr Trp Cys Gln Ser
    610                 615                 620

Val Thr Glu Leu Lys Ala Asp Phe Pro Asp Asn Ile Val Ile Ala Ser
625                 630                 635                 640

Ile Met Cys Ser Tyr Asn Lys Asn Asp Trp Thr Glu Leu Ala Lys Lys
                645                 650                 655

Ser Glu Asp Ser Gly Ala Asp Ala Leu Glu Leu Asn Leu Ser Cys Pro
            660                 665                 670

His Gly Met Gly Glu Arg Gly Met Gly Leu Ala Cys Gly Gln Asp Pro
        675                 680                 685

Glu Leu Val Arg Asn Ile Cys Arg Trp Val Arg Gln Ala Val Gln Ile
    690                 695                 700

Pro Phe Phe Ala Lys Leu Thr Pro Asn Val Thr Asp Ile Val Ser Ile
705                 710                 715                 720

Ala Arg Ala Ala Lys Glu Gly Gly Ala Asn Gly Val Thr Ala Thr Asn
                725                 730                 735

Thr Val Ser Gly Leu Met Gly Leu Lys Ser Asp Gly Thr Pro Trp Pro
            740                 745                 750

Ala Val Gly Ile Ala Lys Arg Thr Thr Tyr Gly Gly Val Ser Gly Thr
        755                 760                 765

Ala Ile Arg Pro Ile Ala Leu Arg Ala Val Thr Ser Ile Ala Arg Ala
    770                 775                 780

Leu Pro Gly Phe Pro Ile Leu Ala Thr Gly Gly Ile Asp Ser Ala Glu
785                 790                 795                 800

Ser Gly Leu Gln Phe Leu His Ser Gly Ala Ser Val Leu Gln Val Cys
                805                 810                 815

Ser Ala Ile Gln Asn Gln Asp Phe Thr Val Ile Glu Asp Tyr Cys Thr
            820                 825                 830

Gly Leu Lys Ala Leu Leu Tyr Leu Lys Ser Ile Glu Glu Leu Gln Asp
        835                 840                 845

Trp Asp Gly Gln Ser Pro Ala Thr Val Ser His Gln Lys Gly Lys Pro
    850                 855                 860

Val Pro Arg Ile Ala Glu Leu Met Asp Lys Lys Leu Pro Ser Phe Gly
865                 870                 875                 880

Pro Tyr Leu Glu Gln Arg Lys Lys Ile Ile Ala Glu Asn Lys Ile Arg
                885                 890                 895

Leu Lys Glu Gln Asn Val Ala Phe Ser Pro Leu Lys Arg Ser Cys Phe
            900                 905                 910

Ile Pro Lys Arg Pro Ile Pro Thr Ile Lys Asp Val Ile Gly Lys Ala
        915                 920                 925

Leu Gln Tyr Leu Gly Thr Phe Gly Glu Leu Ser Asn Val Glu Gln Val
    930                 935                 940

Val Ala Met Ile Asp Glu Glu Met Cys Ile Asn Cys Gly Lys Cys Tyr
945                 950                 955                 960

Met Thr Cys Asn Asp Ser Gly Tyr Gln Ala Ile Gln Phe Asp Pro Glu
                965                 970                 975

Thr His Leu Pro Thr Ile Thr Asp Thr Cys Thr Gly Cys Thr Leu Cys
            980                 985                 990

Leu Ser Val Cys Pro Ile Val Asp  Cys Ile Lys Met Val  Ser Arg Thr
        995                 1000                 1005

Thr Pro  Tyr Glu Pro Lys Arg  Gly Val Pro Leu Ser  Val Asn Pro
    1010                 1015                 1020

Val Cys
    1025

<210>  3
<211>  23
<212>  DNA
<213>  Homo sapiens

<400>  3
ttgcacattg ggtagatgag gac                                              23


<210>  4
<211>  23
<212>  DNA
<213>  Homo sapiens

<400>  4
ttgcacattg ggtaggtgag gac                                              23


<210>  5
<211>  23
<212>  DNA
<213>  Homo sapiens

<400>  5
ctgcacattg ggtagatgag gac                                              23


<210>  6
<211>  23
<212>  DNA
<213>  Homo sapiens

<400>  6
ttgcacattg ggtagatggg gac                                              23


<210>  7
<211>  23
<212>  DNA
<213>  Homo sapiens

<400>  7
ctgcgcatcg ggtagatgag gac                                              23


<210>  8
<211>  23
<212>  DNA
<213>  Homo sapiens

<400>  8
ttgcacattg ggtagatgag aac                                              23


<210>  9
<211>  23
<212>  DNA
<213>  Homo sapiens

<400>  9
ttgcacatcg ggtagatgag gac                                              23

<210>  10
<211>  23
<212>  DNA
<213>  Homo sapiens

<400>  10
ttgcacattg ggaagatgag gac                                                23

<210>  11
<211>  23
<212>  DNA
<213>  Homo sapiens

<400>  11
ctgcacattg ggtagatgag aac                                                23

<210>  12
<211>  23
<212>  DNA
<213>  Homo sapiens

<400>  12
ttgcacattg ggtagatggg aac                                                23

<210>  13
<211>  23
<212>  DNA
<213>  Homo sapiens

<400>  13
ttgcgcatcg ggtagatgag gac                                                23

<210>  14
<211>  23
<212>  DNA
<213>  Homo sapiens

<400>  14
ttgcacattg ggtaggtggg gac                                                23

<210>  15
<211>  23
<212>  DNA
<213>  Homo sapiens

<400>  15
ttgcacattg ggtagacgag gac                                                23

<210>  16
<211>  23
<212>  DNA
<213>  Homo sapiens

<400>  16
ctgcacattg ggtagatcgg gac                                                23

<210> 17
<211> 23
<212> DNA
<213> Homo sapiens

<400> 17
ttgcacattg ggaagatggg gac 23